# EUROPÄISCHE PATENTANMELDUNG

(11) **EP 2 366 354 A2**
(43) Veröffentlichungstag der Anmeldung: **21.09.2011**
(21) Anmeldenummer: 11158254.0
(22) Anmeldetag: 15.03.2011
(51) Int. Cl.: A61B 18/14

(54) **Klinge für chirurgisches Instrument, chirurgische Schere und elektrochirurgisches, bipolares Schneidinstrument für Gewebeeinschnitte mit Vorgerinnung**

(30) Priorität: 15.03.2010 LV 100033; 02.07.2010 EP 10168259
(71) Anmelder: "Golsen Limited", 3041 Limassol (CY); OOO "Novye Energetichskie Technologil", Moskau 107045 (RU)
(72) Erfinder: Die Erfindernennung liegt noch nicht vor
(74) Vertreter: Jeck, Anton

(57) **Zusammenfassung**

Der Gegenstand der Erfindung ist eine Klinge für ein chirurgisches Instrument, die aus einem Stück Einkristall eines harten, rissbeständigen, abnutzungsfesten, porenfreien Materials mit Antihafteigenschaften auf der Basis von teilweise stabilisiertem Zirkoniumdioxid hergestellt wird. Dabei wird das teilweise stabilisierte Zirkoniumdioxid mittels einer gerichteten Kristallisation der Zirkoniumdioxid-Schmelze hergestellt. Das Zirkoniumdioxid enthält eine stabilisierende Komponente. Die Kristallisation erfolgt bei der Bewegung der Schmelze in einem elektromagnetischen HF-Feld von unten nach oben entlang der senkrechten Wachstumsachse des Blocks mit einer Geschwindigkeit von 2-4mm/h im Laufe von 8-15 Stunden und danach mit einer Geschwindigkeit von 8-15mm/h im Laufe von 10-15 Stunden, mit nachfolgendem Glühen des Einkristalls. Dabei weist das Zirkoniumdioxid bei einer Domänengröße von unter 0,2µm ein Monoblock-Oberflächengefüge und eine Phasenhomogenität der Zusammensetzung in Bezug auf den Umfang und die Oberfläche mindestens im Bereich der Schneidkante auf. Für die Anwendung bei der Schere ist die Klinge mit einer Schneidkante versehen, die von der Schnittebene absteht. Es wird die Berührung der Scherenklingen untereinander in der Schnittebene mittels ihrer Stumpfkanten sichergestellt. Beim elektrochirurgischen, bipolaren Schneidinstrument ist die Durchführung einer Vorgerinnung des vorgegebenen Gebiets im Operationsfeld gleichmäßig rings um die Schnittebene sichergestellt, indem die Druckkante des leitfähigen Teils der Instrumentzweigteile profiliert wird.

## Beschreibung

### Gebiet der Technik

Die Erfindung bezieht sich auf medizinische, chirurgische Instrumente und insbesondere auf benutzerfreundliche Klingen mit extra scharfer und extra verschleißfester Schneidkante, mit verbesserten Antihaft-Eigenschaften. Die Erfindung bezieht sich ferner auf Instrumente mit solchen Klingen und insbesondere auf elektrochirurgische Schneidinstrumente, die bei chirurgischen Eingriffen zu mechanischen Einschnitten, Einschnitten mit Blutstillung, zur punktförmigen Gerinnung und Untersuchung von biologischen Geweben auf dem offenen Operationsfeld und bei laparoskopischen Operationen angewendet werden.

### Stand der Technik

Die Schneidteile von chirurgischen Instrumenten arbeiten unter Bedingungen der chemischen und biologischen Einwirkung sowie unter Einwirkung von durch den Widerstand vom Biogewebe verursachten Belastungen. Chirurgische Instrumente umfassen unter anderem solche Instrumente, die zur Durchtrennung von Biogewebe im Kontaktbereich von zwei Klingen, die sich im Schneidbereich gegenläufig bewegen. Dazu zählen z.B. Scheren und insbesondere Guillotinescheren. Bei solchen Instrumenten verursacht der Kontakt der Klingen untereinander mittels ihrer Schneidekanten und an der Seitenfläche der Klingen während des Schneidens erhöhte Reibung, Vergrößerung der Schneidkraft, erhöhte Verformung und Verletzung des geschnittenen, biologischen Gewebes. Bei elektrochirurgischen Schneidinstrumenten werden die Klingen und ihre Schneidkanten zusätzlich der Einwirkung von elektrischen Feldern ausgesetzt. Die Anforderungen an die Klingen werden durch deren Anwendungsbedingungen festgelegt: Festigkeit, Härte, chemische Beständigkeit, biologische Verträglichkeit, Antihafteigenschaften, Rissbeständigkeit, Abnutzungsfestigkeit, hohe Oberflächengüte, scharfe Schneidkanten, hohe Technologiegerechtigkeit bei hoher Werkstoffqualität, Benutzerfreundlichkeit, bequeme Vorsterilisationsreinigung und Sterilisation.

Bekanntlich werden beim Einsatz von elektrochirurgischen Schneidinstrumenten diejenigen bevorzugt, die die Durchtrennung von Biogewebe mit der Gerinnung des Biogewebes in dem zu schneidenden Gebiet sicherstellen. Dabei weist der Arbeitsteil der Instrumente vorwiegend eine Verbundstruktur aus einem leitfähigen Metallteil und einem dielektrischen Teil auf: der Metallteil dieser Struktur wirkt als Elektrode; er sorgt für das Anlegen von elektrischem Strom an das Biogewebe im Schnittbereich, während der dielektrische Teil für die elektrische Isolation von Elektroden sorgt. Ein HF-Strom wird vom Stromerzeuger an die metallischen Teile geleitet. Bei unipolaren, elektrochirurgischen Instrumenten dringt der Strom von der Elektrode ins Biogewebe des Patienten und wird durch ihn geerdet. Dabei kommt in der Kontaktstelle zwischen Elektrode und Biogewebe die Gerinnung der Oberflächenschicht des Biogewebes zustande. Bei bipolaren, elektrochirurgischen Instrumenten fließt der Strom zwischen zwei Elektroden, und zwischen den Kontaktstellen der Elektroden mit dem Biogewebe entstehen Tiefgerinnungsgebiete. Dabei können die Durchtrennung und die Gerinnung des biologischen Gewebes sowohl gleichzeitig als auch zeitlich verschoben erfolgen. Dabei können die Schneidkanten an den Klingen ausgebildet werden, die entweder den Metallteil oder den dielektrischen Teil einer Verbundstruktur im Arbeitsteil des Instruments darstellen.

Je nach Kompliziertheit bei der Ausbildung jenes Teils der Verbundstruktur, der die Schneidkante trägt, werden an die Klingen zusätzliche Anforderungen gestellt: die Möglichkeit der Ausführung von feinen Klingen mit unterschiedlichen Konstruktionsmerkmalen in Mikrongröße, z.B. Aussparungen, Kanäle, Fasen, Rillen, Nuten, Löcher und Schneidkanten mit Mikronstärke.

Aus dem Stand der Technik ist die Anwendung von Klingen aus Nirosta in chirurgischen Instrumenten bekannt.

Die WO 96/27338 beschreibt eine elektrochirurgische, bipolare Schere mit einer Vorausgerinnung der Gewebe. Die Schere enthält zwei untereinander verbundene Branchen. An ihrem distalen Ende sind Schneidelemente, nämlich Elektroden in Form von Klingen aus rostfreiem Stahl, ausgebildet. Als dielektrischer Teil zur Isolation der Elektroden werden darauf aufgetragene Lacke oder Harze verwendet. Die Mängel dieser Schere sind: ein schneller Verschleiß der Schneidkanten der Metallklingen, keine 100%-ige Garantie für eine Gewebegerinnung im Fall einer gegenseitigen Anordnung der Branchen: der Vorlauf der Gerinnung geht mit der Vergrößerung des Branchen-Öffnungswinkels der Schere zurück.

Aus der EP 0853922 B ist eine elektrochirurgische, bipolare Schere bekannt, in der verschiedene Ausbildungsmöglichkeiten der Schere angewendet werden: ein Arbeitsteil mit einer Verbundstoffbranche, ein Arbeitsteil mit zwei Verbundstoffbranchen, ein Arbeitsteil mit zwei Verbundstoffbranchen, die mit hervorragenden, stromleitenden Metallteilen der Elektroden versehen sind. Diese Metallteile sorgen für einen räumlichen Vorlauf der Gerinnungsfront in Bezug auf die Vorwärtsbewegung beim Schneiden über der im Metallteil ausgebildeten Schneidkante. Dieser Aufbau der Klinge macht den Vorlauf der Gerinnungsfront vom Branchenöffnungswinkel der Schere abhängig: der Vorlauf der Gerinnung geht mit der Vergrößerung des Öffnungswinkels zurück.

Jedoch stumpfen die Metallklingen sehr schnell ab. Das geschieht sowohl bei mehrfachen Sterilisationen unter Einfluss von Hochtemperaturen und chemischen Substanzen als auch beim Kontakt mit dem Biogewebe. Für komplizierte Schnitte müssen während einer Operation mehrere Schneidinstrumente benutzt werden. Dabei können Unterbrechungen während der Operation die Änderung der taktilen Wahrnehmung des Chirurgen sowie eine ungewünschte Durchtrennung der Gewebe verursachen.

Darüber hinaus ist nichtrostender Stahl mit biologischen Geweben schlecht verträglich. Deswegen können im Schnittbereich Mikrothromben entstehen. Sie führen eine Verstopfung von kleinen Blutgefäßen herbei. Dabei ist die Abstumpfung der Stahlklingen ein physikalisch-chemischer Vorgang. Als Folge dringen sowohl Mikro- und Makroteilchen des Metalls als auch die Produkte der chemischen Reaktion zwischen Metall und der aggressiven Umgebung wie Lymphe und Blut in den menschlichen Körper ein. Das kann postoperative Komplikationen verursachen.

Darüber hinaus erlaubt die physikalische Struktur des nichtrostenden Stahls nicht, eine solche Schärfe der Schneidkante zu erreichen, die für die Ausführung von guten Einschnitten erforderlich ist. Die mechanische Behandlung ermöglicht nur eine sägezahnförmige Schneide. Das oben Dargelegte führt zu wesentlichen Verformungen und Verletzungen der Biogewebe, zur Quetschung des Biogewebes und a.m. Dabei verfügt nichtrostender Stahl über keine Antihafteigenschaften. Deswegen haften die Zersetzungsprodukte des Biogewebes an der Klinge. Das erschwert die Arbeit während der Operationen und erfordert auch mehr Zeit während der Vorsterilisationsreinigung sowie bei der Sterilisation. Außerdem sind solche Klingen leitfähig und speichern elektrostatische Ladung, die beim Kontakt zwischen Klinge und Biogewebe über den Körper des Patienten geerdet wird.

Aus dem Stand der Technik sind Klingen aus dielektrischen Stoffen, z.B. aus Keramik, bekannt. Sie beseitigen manche Mängel der Metallklingen und weisen dabei eine hohe Härte auf.

Die RU 2053718 C beschreibt z.B. eine spitzenförmige Klinge mit einem Anschliffwinkel. Die Klinge wird im Griff des chirurgischen Instruments befestigt. Die Klinge wird aus polykristalliner Keramik auf der Basis von alpha- Aluminiumoxid mit Korngefüge und mit einer durchschnittlichen Korngröße von 0,1-3,0µm ausgebildet.

Die WO 92/22257 offenbart ein bipolares, elektrochirurgisches, endoskopisches Instrument mit bipolaren Elektroden an den beweglichen Teilen des Schneidinstruments. Die Elektroden sorgen für eine Blutstillung. Gemäß einer bevorzugten Ausbildung des Instruments werden die Schneidklingen aus einem keramischen Material, z.B. aus Keramik auf der Basis von Zirkonium- oder Aluminiumoxid mit leitfähiger Beschichtung, gefertigt.

Jedoch weisen die bekannten, keramischen Materialien ein körniges Gefüge und eine Porosität auf und werden vorwiegend mittels Sinterns gefertigt. Deswegen können daraus Klingen mit sehr kleiner Stärke praktisch überhaupt nicht hergestellt werden. Aus diesem Grund werden dabei Technologien verwendet, bei denen filmartige keramische Beschichtungen erzeugt werden. Hier wirken auf einem Träger ausgebildete Beschichtungskanten als Schneidkanten.

Die bekannte, elektrochirurgische, bipolare Schere (Modell Powerstar BP100 der Fa. Eticon, USA) besteht aus gekreuzten, isolierten Zweigteilen, die mittels eines elektrisch isolierten Gelenks verbunden werden. Das eine Zweigteil weist eine Verbundstruktur Metall-Dielektrikum auf. Die dielektrische Klinge stellt eine keramische Schicht dar, die auf den Innenteil eines Zweigteils aufgetragen wird. Die zweite Klinge auf dem anderen Zweigteil besteht aus Metall. Jedoch erfolgen die Gerinnung und die Durchtrennung gleichzeitig mit dem Schneiden. Das vermindert wesentlich die Wirksamkeit der Blutstillung während der Durchtrennung. Dabei wird keine Symmetrie der Gerinnungszonen beidseitig von der Schnittebene sichergestellt. Die Ausbildung der Klingen aus ungleichartigen Metallen im Falle der Guillotinenschere bedingt eine erhöhte Reibung während der Ausführung der Zerschneidungen, eine Vergrößerung der Schneidkraft, eine erhöhte Verformung und Verletzung des geschnittenen, biologischen Gewebes.

Bei manchen bekannten, elektrochirurgischen, bipolaren Scheren (US 5 324 289 B1, US 5810808 B1, US 5342381 B1, US 5779701) wird vorgeschlagen, die Schneidkante der Klingen auf dem dielektrischen Teil zu platzieren. Dabei kann der dielektrische Teil der Verbundklingen aus Glas oder Bornitrid oder aus Industriediamant oder aus Aluminiumoxid-Keramik, Zirkoniumdioxid-, Titandioxid- oder Chromdioxid-Keramik gebildet werden. Die dielektrische Schicht kann auf den Metallteil der Verbundstruktur mittels Thermalpräzipitation oder Aufspritzen aufgetragen werden.

Die Verwendung von Glasklingen wird wegen der zu niedrigen Rissbeständigkeit von Glas verhindert, denn dabei entstehen Ausbrüche an der Schneidkante während der Operation.

Wenn keramische Klingen eingesetzt werden, sowohl plattenförmig als auch in Form von Beschichtungen, so trägt ihr poröses, körniges Gefüge zum beschleunigten Abbau der Schneidkante bei, der auch infolge der Spannungsdurchschläge im Bereich der Schneidkante erfolgt. Darüber hinaus trägt dies zur Krustenbildung auf den Schneidebenen der Klingen bei, erschwert den Ablauf bei der Vorsterilisationsreinigung und der Sterilisation. Die Anschliffschärfe der Schneidkante ist durch die Größe der Sinterkörner begrenzt. Es ist so gut wie unmöglich, eine abnutzungsfeste Schneidkante zu gewinnen, deren Korngröße unter der maximalen Korngröße liegt.

Eines der aussichtsreichen Materialien zur Herstellung von Klingen für chirurgische Instrumente ist der Werkstoff auf der Basis von teilweise stabilisiertem Zirkoniumdioxid mit stabilisierender Beimischung von Yttriumoxid. Dieses Material wird mittels gezielter Kristallisation der Schmelze in einem kalten Behälter hergestellt. Die Schmelze enthält z.B. 97 Mol-% ZrO₂ und 3 Mol-% Y₂O₃ und ist durch eine Biegefestigkeit und Druckfestigkeit von 500-800 MPa und eine4 Bruchzähigkeit (Spannungsintensitätsfaktor) von 5-16 MPa/m^{1/2} gekennzeichnet (G.A. Gogotsi, E.E. Lomonova, V.G. Peichev "Strength and Fracture Toughness of Zirconia Crystals"., J. Eur. Ceram. Soc., V. 11, S. 123-132, 1993). Jedoch weist dieses Material eine ungenügend hohe Abnutzungsfestigkeit auf, die mit der verhältnismäßig niedrigen Biegefestigkeit zusammenhängt. Dieses Material hat auch einen großen Streubereich der Bruchzähigkeit, der mit der Inhomogenität dieses Materials in Bezug auf die Mikrostruktur und Phasenzusammensetzung zusammenhängt.

Eines der aussichtsreichen, dielektrischen Materialien für Schneidklingen ist der Werkstoff auf der Basis von Zirkoniumdioxid, teilweise stabilisiertem Zirkoniumdioxid (oder PSZ). Dieser Werkstoff wird mittels einer gerichteten Kristallisation der Schmelze von Zirkoniumdioxid mit einer stabilisierenden Beimischung, wie z.B. Yttriumoxid, in einem kalten Behälter hergestellt (M.A. Borik, M.A. Vischnyakova, O.M. Zhigalina, A.B. Kulebyakin, S.V. Lavrishshev, E.E. Lomonosova, V.V. Osiko "Forschung der Mikro- und Nanogefüge der Kristalle des teilweise stabilisierten Zirkoniumdioxids". Nanotechnologien von Russland, 2008, 3, Heft 11-12, S. 76-81; Yu.S. Kuzminov, E.E. Lomonosova, V.V. Osiko, "Schwerflüssige Materialien aus Kalttiegel", Moskau, NAUKA, 2004; 2. Yu.S. Kuzminov, E.E. Lomonova and V.V. Osiko "Cubic zirconia and Skull Meeting", Cambridge, UK. 346 S. 2008).

Kristalle des teilweise stabilisierten Zirkoniumdioxids erhalten ihre Funktionsfähigkeit im Temperaturbereich von -140°C bis 1400°C. Gegenüber anderen Keramiken weisen diese Kristalle die höchste Bruchzähigkeit und eine höhere Abnutzungsfestigkeit auf. Die Kristalle werden mittels gerichteter Kristallisation der Zirkoniumdioxid-Schmelze bei einer Wachstumsgeschwindigkeit von 3-40mm/St. gezüchtet. Die Zirkoniumdioxid-Schmelze enthält eine stabilisierende Komponente, z.B. Yttriumoxid. Im Unterschied zur üblichen Oxidkeramik (die mittels Sintern von Ausgangs-Nanopulvern gefertigt wird) wird das teilweise stabilisierte Zirkoniumdioxid infolge der Selbstorganisation des Nanogefüges während des kubisch-quadratischen Phasenübergangs der Monokristalle gebildet. Als Ergebnis wird der Stoffumfang aus den dicht und geordnet gepackten Domänen mit typischen Größen von 10-20nm zusammengesetzt. Dank ihres einzigartigen Gefüges weisen die Kristalle des teilweise stabilisierten Zirkoniumdioxids außerordentlich hohe mechanische und physikalischchemische Eigenschaften auf: eine hohe Schlagzähigkeit und Abnutzungsfestigkeit in Kombination mit einer niedrigen Reibungszahl, eine hohe Wärmebeständigkeit, biologische Inaktivität und biologische Verträglichkeit.

Aus der RU 2220674 C1 ist ein chirurgisches Instrument bekannt, dessen Klinge aus einem Material auf der Basis von Zirkoniumdioxid aus einem Kristall gebildet ist. Der Kristall weist ein nanokristallines Gefüge mit einer Domänengröße von 200nm ohne Phasengrenzen auf. Der Kristall ist mittels Yttriumoxid und zusätzlich mittels einer anderen Komponente stabilisiert. Als zusätzliche Stabilisationskomponente wird ein Metalloxid verwendet, das aus folgender Gruppe gewählt wird: Kalziumoxid, Magnesiumoxid, Strontiumoxid, Skandiumoxid und Oxide der Seltenerdmetalle von Europium bis Lutetium. Das Komponentenverhältnis ist in Mol-% wie folgt: Hauptstabilisationskomponente Yttriumoxid 0,5-4,5, zusätzliche Stabilisationskomponente 0,1- 4,5, Der Rest aus Zirkoniumoxid. Dabei ist die Klingenbreite 1-15mm, die Klingenstärke 0,15-1mm, die Länge der Schneidkante 1-60mm und der Anschliffradius der Schneidkante der Klinge 0,05-2,0µm. Dabei weist die Klinge einen Doppelanschliffwinkel auf: einen Hilfsanschliffwinkel von 15-25° und einen Hauptanschliffwinkel der Schneidkante von 30-60°. Die Arbeitsflächenbreite der Schneidkante des Hauptanschliffwinkels ist 30-500µm. Das Schneidkantenprofil der Klinge kann gerade, abgerundet, kombiniert und zweischneidig sein.

Die aus einem Kristall mittels mechanischer Bearbeitung (Schneiden, Schleifen, Polieren) gebildeten Klingen haben auf der Klingenoberfläche eine mechanisch gespannte Schicht. Das verursacht eine gefügemäßig inhomogene Oberfläche (Gruppenbildung) und löst auch einen tetragonal-monoklinen Phasenübergang in lokalen Stellen der bearbeiteten Oberfläche aus. Diese Gefügeinhomogenität und die Einschlüsse der monoklinen Phase verringern die Rissbeständigkeit und die Abnutzungsfestigkeit des Materials, weil sie die Zerstörung des Instruments bei seiner Benutzung und Sterilisation verursachen.

### Offenbarung der Erfindung

Die Aufgabe der Erfindung ist die Entwicklung einer Klinge für ein chirurgisches Instrument. Die Klinge sollte einen sehr kleinen Anschliffradius der Schneidekante aus porenfreiem, stabilisiertem Zirkoniumdioxid mit erhöhter Rissbeständigkeit, Festigkeit und Abnutzungsfestigkeit haben. Bei hohen, physikalisch-chemischen Eigenschaften sollte diese Klinge eine Phasen- und Gefügehomogenität des Materials im Bereich der Schneidkanten sowie über ihre Länge aufweisen, und zwar auch nach der Ausbildung der Klingen anhand mechanischer oder anderer Bearbeitungsverfahren, die mit mechanischer Einwirkung und mit ungleichmäßiger Erhitzung des Klingenmaterials verbunden sind. Diese Einwirkungen ergeben eine gefügemäßige Inhomogenität und eine Phasentransformation des Zirkoniumdioxids. Das sollte es ermöglichen, schärfere Schneidkanten dank der Benutzung der Materialstrukturbesonderheiten zu benutzen. Diese Struktur ist durch den Züchtungsvorgang des genannten Materials sowie durch das Fehlen von jeglichen Korngrenzen und Poren im Umfang und auf der Oberfläche des Materials bedingt.

Darüber hinaus ist die weitere Aufgabe der Erfindung die Entwicklung eines elektrochirurgischen Schneideinstruments, bei dem optimale Bedingungen für eine Vorgerinnung innerhalb der vorgegebenen Zeit bis zur Durchtrennung des Biogewebes geschaffen werden müssen. Dabei soll eine für Gewebeeinschnitte optimale Gestaltung des Schneidenteils des Instruments verwendet werden. Diese Gestaltung wird durch die physikalisch-chemischen Eigenschaften der Klingen festgelegt.

Bei der Ausarbeitung der Erfindung wurden unterschiedliche Werkstoffe auf der Basis von teilweise stabilisiertem Zirkoniumdioxid erforscht. Diese Werkstoffe wurden mittels gerichteter Kristallisation aus der Schmelze von Zirkoniumdioxid mit einer stabilisierenden Komponente erzeugt.

Dabei war bekannt, dass das reine Zirkoniumdioxid in drei Modifikationen existieren kann: monoklin, tetragonal und kubisch. Die monokline Niedertemperatur-Modifikation ist bis zur Temperatur von 1160°C stabil. Im Temperaturbereich von 1160-2370°C ist die tetragonale Modifikation beständig. Bei einer Temperatur von über 2370° und bis zur Schmelztemperatur (2680°C) ist die kubische Modifikation beständig.

Die Neigung von Zirkoniumdioxid zur Bildung von Festlösungen mit Oxiden der Elemente aus der 2. und der 3. Gruppe des Periodensystems, Z.B- Y₂O₃, Ln₂O₃, (wobei Ln für alle Seltenerdmetalle steht) ist gut bekannt. Dabei ist von Bedeutung, dass bei der Bildung von Festlösungen die Temperaturen beider Phasenübergänge von ZrO₂ wesentlich zurückgehen. Das verschafft die Möglichkeit, die kubische Hochtemperatur-Modifkation von ZrO₂ kinetisch zu stabilisieren.

Die Forschungen über das teilweise stabilisierte Zirkoniumdioxid haben gezeigt, dass bei der gerichteten Kristallisation der Schmelzen mit einer Zusammensetzung von ZrO₂ und 2-6 Mol-% Y₂O₃ Kristalle der kubischen Modifikation gebildet werden. Mit dem Vorrücken der Temperaturfront und der allmählichen Abkühlung der gezüchteten Kristalle bis zur Überganstemperatur von kubisch nach tetragonal (2400-2300°C je nach der Konzentration des Stabilisationsoxids) bleiben die Kristalle phasenmäßig homogen und kubisch. Bei weiterer, langsamer Abkühlung erfolgt der Übergang von der kubischen in die Tetragonalstruktur. Diese Struktur bleibt bei der Abkühlung bis zur Raumtemperatur erhalten.

Die hergestellten Kristalle haben eine glatte Oberfläche. Sie sind milchweiß. Das ist das äußere Merkmal des darin erfolgten Phasenübergangs. Die Kristalle erhalten ihre Kontinuität. Sie haben keine Risse. Die einzelnen Kristalle in der Gruppe der gezüchteten Kristalle sind dicht aneinander im Blockumfang gepackt. Es sind keine Abstände zwischen den Kristallen vorhanden.

Der Umwandlung des Festlösungszustands liegen zwei Vorgänge zugrunde. Dabei können sich die Vorgänge je nach Zusammensetzung der Festlösung (Art der Stabilisationsoxids und seiner Konzentration) und den Herstellungsbedingungen sowohl in reiner Form als auch in unterschiedlichen Kombinationen zeigen:
1) Diffusionsverfahren: dabei kommt die Zersetzung der Festlösung zustande; dabei entstehen im Umfang der ursprünglichen, kubischen Festlösung Herde der Tetragonalphase, die durch das Stabilisationsoxid abgemagert ist; die kubische Matrix wird nun durch den Stabilisator angereichert und vollführt den Übergang in die Tetragonalphase bei niedrigeren Temperaturen; im Temperaturbereich von 1000-1200°C wird die Diffusion erschwert, Deswegen wird dieser Mechanismustyp abgestellt; die chemische Zusammensetzung der sich bildenden Domänen ist der Zusammensetzung der ursprünglichen kubischen Phase ähnlich, und
2) diffusionsloses Verfahren: dabei führt die Tetragonalphase bei Temperaturen von unter 1200°C zur Bildung von Domänen dieser Phase.

Der kubisch-tetragonale Phasenübergang im System ZrO₂ und 2,5-4 Mol-% Y₂O₃ erfolgt während der Abkühlung der gezüchteten Kristalle. Als Ergebnis entsteht ein hierarchisches Mikro- und Nanogefüge. Es schließt geordnete Gebiete einer parkettweisen Verpackung oder von Plattenstapeln ein. Die Größe der Domänen des 1. Niveaus beträgt ca. 10nm. Die geordneten Gebiete wechseln die Gebiete ab, die als chaotisch angeordnete Nanodomänen unterschiedlicher Form, wie Prismen, Platten oder Nadeln, zusammengesetzt sind. Mit der Steigerung der Yttriumoxid-Konzentration wird das wenig geordnete und mit Rissen durchdrungene Mikrogefüge allmählich in ein geordnetes Tweed-Gefüge umgewandelt, ohne dass die Kontinuität gestört wird. Die Oberflächenbeschaffenheit korreliert mit dem räumlichen Nanogefüge. Somit gilt das Oberflächenmuster der Kristalle des teilweise stabilisierten Zirkoniumdioxids als gutes Diagnosezeichen für ein räumliches Nanogefüge.

Das einzigartige Nanodomänen-Gefüge des teilweise stabilisierten Zirkoniumdioxids ist außerordentlich plastisch. Es stellt solche Eigenschaften der Kristalle sicher, die sich von den Eigenschaften der Sinterkeramik mit jeweiliger Zusammensetzung unterscheiden:
- die höchste Dichte des teilweise stabilisierten Zirkoniumdioxids für die genannte Zusammensetzung; das ist die Folge der Aufrechterhaltung der Kontinuität und der vollständigen Porenfreiheit;
- eine hohe Festigkeit, die die Festigkeit von Sinterkeramik überschreitet;
- eine Rissbeständigkeit, die die von Sinterkeramik und anderen Nichtmetallmaterialien überschreitet;
- hohe, tribotechnische Eigenschaften: eine sehr niedrige Reibungszahl und eine hohe Verschleißfestigkeit;
- die Fähigkeit zur Aufrechterhaltung hoher, mechanischer Eigenschaften innerhalb eines großen Temperaturbereichs von -140°C bis 1400°C;
- eine chemische Inaktivität und biologische Verträglichkeit (V.V. Osiko "Extrastrong wear-resistant materials based on nanostructured crystals of partially stabilized zirconium dioxide", Mendeleev Commun., 2009, 19, S. 117-122).

Während der Forschungen über das teilweise stabilisierte Zirkoniumdioxid (PSZ) wurde festgestellt, dass das Gefüge des teilweise stabilisierten Zirkoniumdioxids von der Zusammensetzung der Schmelze und insbesondere von der Art und der Konzentration der Stabilisationskomponenten der Festlösung, des Temperaturverlaufs während der Bildung und der zusätzlichen Wärmebehandlung abhängig ist.

Es wurde das teilweise stabilisierte Zirkoniumdioxid erforscht. Das teilweise stabilisierte Zirkoniumdioxid wurde als ein Block mit einem Durchmesser von 400mm und einer Höhe von 180mm nach dem Verfahren der gerichteten Kristallisation der Dioxid-Schmelze hergestellt. Die Dioxid-Schmelze enthielt 0,2-8,0 Mol-% Yttriumoxid. Die Schmelze wurde in einem elektromagnetischen HF-Feld entlang der senkrechten Wachstumsachse mit einer Geschwindigkeit von 2- 4mm/h im Laufe von 8-15 Stunden und danach mit einer Geschwindigkeit von 8- 15mm/h im Laufe von 10-15 Stunden von oben nach unten bewegt. Die Schmelze wurde auch in der Luft bei einer Temperatur von 1250-1400°C im Laufe von 10- 100 Stunden geglüht oder in verdünnter Luft bei einem Druck von 10-10⁻¹mm Hg und bei einer Temperatur von 2000-2200°C im Laufe von 2-10 Stunden geglüht. Infolge dieser Forschungen haben die Erfinder Folgendes festgestellt:
A) die Züchtung von einem Kristallblock mit geringer Geschwindigkeit ermöglicht es in der Anfangsstufe, die Kristallanzahl im Block zu vermindern und die Abmessungen einzelner Kristalle zu vergrößern; durch die nachfolgende Steigerung der Wachstumsgeschwindigkeit wird die mehr homogene, phasenmäßige Zusammensetzung im Umfang der Kristalle erreicht, und zwar aufgrund der kürzeren Verweilzeit seiner einzelnen Teile in verschiedenen Temperaturzonen beim Wachstum.
B) die oben genannten Eigenschaften der teilweise stabilisierten Zirkoniumdioxide werden durch Prüfungen nachgewiesen; sie sind durch folgende Faktoren bedingt: strukturelle Vollkommenheit der Interdomänen-Grenzen in Kristallen der teilweise stabilisierten Zirkoniumdioxide, Homogenität bei der Verteilung der Festlösungskomponenten, Homogenität der phasenmäßigen Zusammensetzung im Kristallumfang und Porenfreiheit; die Vergleichseigenschaften der teilweise stabilisierten Zirkoniumdioxide mit Keramik auf der Basis von Aluminiumoxid und von Zirkoniumdioxid sind der Tabelle unten zu entnehmen.

**Tabelle**

| Material | Mikro-Härte, GPa | Biegefestigkeit, MPa | Bruchzähigkeit, MPa*m^{0,5} | Eigenkorngröße, µm |
|---|---|---|---|---|
| Teilweise stabilisiertes Zirkoniumdioxid, hergestellt nach dem oben genannten Verfahren | 15,08 | 800-1200 | 10 | nicht vorhanden |
| Keramik auf Basis von Al₂O₃ | 13,5 | 65-800 | 5,2-8,0 | 1,0-5,0 |
| Keramik auf Basis von ZrO₂ | 12,8 | 600- 800 | 5,8-9,0 | 1,0 - 5,0 |

B) Bei allen erforschten Proben, unabhängig vom Anteil der Sterilisationsbeimischung, wird das hergestellte, teilweise stabilisierte Zirkoniumdioxid folgendermaßen charakterisiert: Einkristall mit großoberflächigem Domänen-Zwillingsgefüge mit zwei der kubischen Phase nahen Tetragonalmodifikationen des Zirkoniumdioxids mit einem unterschiedlichen Grad an Tetragonalität: 1,005-1,007 und 1.014-1,035; die Dopplungsebene (Verzwilligungsebene) der Domänen ist Ebene (110).

Dabei weist das Gebiet der primären Verzwilligung eine Plattenform auf. Diese Platten verzwilligen sich ihrerseits und bilden somit ein parkettweises Gefüge aus Zwillingsdomänen. Die Spuren der sekundären Verzwilligungsebene liegen unter einem Winkel von etwa 45° zur Ebene der primären Verzwilligung. Die Dopplung erfolgt in den Ebenen, die zur vierzähligen Symmetrieachse der ursprünglichen Hochtemperaturphase geneigt sind. Es wird die Verzwilligungshierarchie beobachtet: es gibt die Zwillinge der 1., der 2., der 3. Folge usw. Dabei enthält jeder der Zwillinge die Zwillinge der nächsten Folge in sich.

Ein solches Gefüge führt im Endeffekt zur Nanostrukturierung des Materials mit der Bildung von Domänengefügen, deren Größen unter 0,2µm liegen. Somit kann das Material des teilweise stabilisierten Zirkoniumdioxids je nach Vorhandensein des oben beschriebenen Zwillingsgefüges nach dem Verfahren der Durchstrahlungselektronenmikroskopie identifiziert werden.

Infolge der Forschungen wurde festgestellt, dass die Relaxation der elastischen Spannungen nicht durch die Bildung von Versetzungen, sondern durch die Verzwilligung erfolgte. Der Anteil einer Sterilisationsbeimischung beeinflusst nicht monoton das Gefüge. Die Mindestgrößen der Zwillinge entsprechen der Konzentration von 3,2 Mol-% Y₂O₃. Es ist der Optimalbereich von 2,8-3,7 Mol-% Y₂O₃ gewählt worden. Er ist durch hohe mechanische Eigenschaften gekennzeichnet. Innerhalb dieses Bereichs wird der Charakter des Nanogefüges aufrechterhalten. Es werden nur die Domänengrößen geändert.

Gleich große Plattenprobestücke wurden aus einem Einkristall des teilweise stabilisierten Zirkoniumdioxids entlang der Wachstumsachse oder chaotisch ausgeschnitten. Sie wurden einer tiefgreifenden, mechanischen Einwirkung ausgesetzt, die mit dem inhomogenen Temperaturverlauf im Umfang und auf der Oberfläche zusammenhängen, z.B. Schleifen, Schneiden, Polieren, mechanische Zerkleinerung bis zum Pulver mit einer Korngröße von etwa 50-150µm. Bei solchen Plattenprobestücken liegen Änderungen der Phasenzusammensetzung der Kristalle vor. Sie hängen mit dem Übergang der transformierbaren Tetragonalphase in die monokline Phase zusammen. Während der mechanischen Bearbeitung wird die Oberflächenschicht gestört. Die darin entstehenden Spannungen verursachen die Bildung von Gefügeinhomogenitäten, die mit Domänendrehungen und der Blockbildung auf der verarbeitbaren Oberfläche zusammenhängen. Die entstehende Gefüge- und Phaseninhomogenität der Oberfläche nach der mechanischen Bearbeitung trägt zur Bildung von lokalen Spannungen bei, die Oberffächen-Mikrorisse verursachen. Diese Mikrorisse vermindern die Rissbeständigkeit und die Abnutzungsfestigkeit des Materials.

Dabei haben die Erfinder Folgendes festgestellt: um das teilweise stabilisierte Zirkoniumdioxid als Konstruktionswerkstoff zur Fertigung der Klingen für medizinische Instrumente benutzen zu können, ist es wichtig, die Richtung der Anordnung der am meisten beanspruchten Teile der Klingen, und zwar der Schneidekanten entlang der kristallographischen Achsen <100> des Einkristallgitters des teilweise stabilisierten Zirkoniumdioxids zu berücksichtigen. Es ist auch wichtig, die Wiederherstellung der Phasenhomogenität des Materials nach der mechanischen Behandlung sicherzustellen.

Die gestellte Aufgabe wurde mittels Herstellung einer Klinge für das chirurgische Instrument gelöst. Die Klinge wird aus einem dielektrischen Material gebildet und mit der Schneidkante versehen. Die Klinge zeichnet sich dadurch aus, dass sie aus einem Stück Einkristall eines harten, rissbeständigen, abnutzungsfesten, porenfreien Materials mit Antihafteigenschaften auf der Basis des teilweise stabilisierten Zirkoniumdioxids gebildet wird. Dabei wird das teilweise stabilisierte Zirkoniumdioxid mittels gerichteter Kristallisation der Zirkoniumdioxid-Schmelze hergestellt. Die Zirkoniumdioxid-Schmelze enthält eine stabilisierende Komponente. Bei einer Domänengröße von unter 0,2µm weist die Zirkoniumdioxid-Schmelze ein Monoblock-Oberflächengefüge und die Phasenhomogenität der Zusammensetzung in Bezug auf den Umfang und die Oberfläche mindestens im Bereich der Schneidekante auf.

Dabei ist es erfindungsgemäß zweckmäßig, dass die Schneidkante der genannten Klinge mit der Ausrichtung entlang einer der kristallographischen Achsen <100> des Einkristallgitters gebildet ist.

Dabei kann die Schneidkante erfindungsgemäß geradlinig ausgebildet werden.

Dabei ist es erfindungsgemäß zweckmäßig, dass die Klinge aus einem Stück Einkristall des stabilisierten Zirkoniumdioxids gebildet ist. Dieses stabilisierte Zirkoniumdioxid wird mittels einer gerichteten Kristallisation der Schmelze hergestellt. Dafür wird die Schmelze in einem elektromagnetischen HF-Feld von unten nach oben entlang der senkrechten Wachstumsachse mit einer Geschwindigkeit von 2-4mm/h im Laufe von 8-15 Stunden und danach mit einer Geschwindigkeit von 8-15mm/h im Laufe von 10-15 Stunden bewegt. Danach erfolgt das Glühen des Einkristalls, wodurch die Phasen- und die Gefügehomogenität seines Umfangs und seiner Oberfläche sichergestellt wird.

Dabei ist es erfindungsgemäß zweckmäßig, dass die tetragonale Phasenhomogenität des Umfangs und der Oberfläche des Einkristalls sichergestellt wird.

Dabei kann die genannte tetragonale Phasenhomogenität im Umfang und auf der Oberfläche des Einkristalls durch das Glühen des Einkristalsl in der Luft bei einer Temperatur von 1250-1400°C im Laufe von 10-100 Stunden erreicht werden.

Dabei können die tetragonale Phasenhomogenität im Einkristallumfang und die Kontrastfärbung des Einkristalls erfindungsgemäß mittels Glühen des Einkristalls bei einer Temperatur von 2000-2200°C in verdünnter Luft bei einem Druck von 10⁻²-10⁻⁴mm Hg im Laufe von 2-10 Stunden sichergestellt werden.

Dabei kann als Stabilisationskomponente erfindungsgemäß Yttriumoxid verwendet werden.

Dabei kann die Klinge erfindungsgemäß aus einem Stück Einkristall des genannten, stabilisierten Zirkoniumdioxids gefertigt werden. Dabei wird das stabilisierte Zirkoniumdioxid aus der Schmelze von Zirkoniumdioxid mit einem Yttriumoxid-Anteil von 0,2-8,0 Mol-% produziert.

Dabei kann die Klinge erfindungsgemäß aus einem Stück Einkristall des genannten Zirkoniumdioxids gefertigt und mittels Oxiden der Seltenerdmetalle stabilisiert werden. Dazu zählen Elemente von Zer bis Lutetium.

Dabei kann die Klinge erfindungsgemäß aus einem Stück Einkristall des genannten Zirkoniumdioxids gefertigt werden. Das Zirkoniumdioxid wird aus der Schmelze produziert, die 0,1-5,0 Mol-% der stabilisierenden Oxide der Seltenerdmetalle enthält. Dabei kann die Klinge erfindungsgemäß aus einem Stück Einkristall des genannten Zirkoniumdioxids gefertigt werden. Das Zirkoniumdioxid wird aus der Schmelze hergestellt, die solche Beimischungen enthält, die den Färbungskontrast der Klinge im Hintergrund des Operationsfelds sicherstellen.

Dabei kann die Klinge erfindungsgemäß aus einem Stück Einkristall des genannten Zirkoniumdioxids gefertigt werden. Das Zirkoniumdioxid wird aus der Schmelze produziert, die 0,1-5,0 Mol-% der stabilisierenden Oxide der Seltenerdmetalle enthält, die die Kontrastfärbung des Klingenmaterials im Hintergrund des Operationsfelds sicherstellen.

Dabei ist es erfindungsgemäß zweckmäßig, dass die Klinge aus einem Stück Einkristall des stabilisierten Zirkoniumdioxids gebildet ist. Das Zirkoniumdioxid enthält untereinander ausgerichtete Zwillingsgefüge unter einem Winkel von 45° zur Ebene der Verzwilligung. Sie werden durch tetragonale Phasen mit unterschiedlichem Grad der Tetragonalität gebildet. Ihre Tetragonalitätsachsen sind zueinander unter einem Winkel von 85-90° ausgerichtet und sind nicht kollinear. Dabei weist die Klinge mindestens im Bereich der Schneidkante eine tetragonale Phasenhomogenität auf.

Dabei ist es erfindungsgemäß zweckmäßig, dass die Klinge aus einem Stück Einkristall des stabilisierten Zirkoniumdioxids gebildet ist. Das Zirkoniumdioxid enthält Zwillingsgefüge, die mittels Kristallen der Tetragonalphasen gebildet sind. Der Tetragonalitätsgrad beträgt 1,005-1,007 und 1,014-1,035.

Dabei kann die Klinge erfindungsgemäß aus einem Stück Einkristall des genannten, stabilisierten Zirkoniumdioxids gefertigt werden. Dieses stabilisierte Zirkoniumdioxid wird aus einer Zirkoniumdioxidschmelze mit 2,8-3,7 Mol-% Yttriumoxid hergestellt. Dabei kann die genannte, tetragonale Phasenhomogenität erfindungsgemäß mittels Glühen der fertigen Klinge mindestens im Bereich der Schneidkante der fertigen Klinge sichergestellt werden.

Dabei kann die Klinge erfindungsgemäß in der Luft geglüht werden, und zwar wird die Klinge im Laufe von 2-5 Stunden bei einer Temperatur von 1200-1350°C mit einer Temperaturzunahmegeschwindigkeit von 6-10°C/min und einer Temperaturabnahmegeschwindigkeit von 6-8°C/min gehalten.

Außerdem sollte die Schneidkante erfindungsgemäß und zweckmäßig 0,2-0,5µm breit sein.

Dabei ist es erfindungsgemäß zweckmäßig, dass die Schneidkante geradlinig ausgebildet ist.

Dabei kann die Klinge erfindungsgemäß für die Anwendung in Form eines Belags oder eines Einsatzelements (Einschubstücks) für eine Gruppe von chirurgischen Instrumenten angepasst werden. Diese Gruppe umfasst das Skalpell, das Schneidmesser, Scheren, elektrochirurgische, unipolare Schneidinstrumente und elektrochirurgische, bipolare Schneidinstrumente.

Dabei kann die Klinge erfindungsgemäß für die Anwendung in Form eines Einsatzelements für chirurgische Scheren angepasst werden. Die Klinge wird als Platte mit kleiner Trapezform im Querschnitt ausgebildet. Die Platte wird über ihre gesamte Länge mit einer Fase geneigt zur vermutlichen Schneidebene versehen. Die Fase stellt die Ausbildung der Schneidekante und der davon abstehenden Stumpfkante der Berührungslinie der Instrumentklingen untereinander in der Schnittebene sicher. Dabei kann die Klinge erfindungsgemäß für ihre Benutzung im elektrochirurgischen, bipolaren Schneidinstrument angepasst werden. Die Klinge wird als Platte mit kleiner Trapezform im Querschnitt ausgebildet. Diese Platte wird über ihre gesamte Länge mit einer Fase, geneigt zur vermutlichen Schneideebene, versehen. Die Fase stellt die Ausbildung der Schneidkante und der davon abstehenden Stumpfkante der Berührungslinie der Instrumentklingen untereinander in der Schnittebene sicher.

Die gestellte Aufgabe wurde auch dadurch gelöst, dass eine chirurgische Schere mit zwei Zweigteilen entwickelt wurde. Die Zweigteile sind beweglich mittels eines Gelenks verbunden. An ihren distalen Enden sind Einsatzelemente in Form von Klingen angeordnet. Die Klingen werden aus dem Material nach einer der oben beschriebenen Ausführungsform hergestellt. Die Gestaltung dieser Klingen stellt die Durchtrennung des Biogewebes im Bereich neben der Schnittebene sicher. Der Klingenschluss erfolgt über die Stumpfkanten in der Schnittebene.

Die gestellte Aufgabe wird auch dadurch gelöst, dass ein elektrochirurgisches, bipolares Schneidinstrument entwickelt wird. Es enthält zwei isolierte, leitfähige Zweigteile. Die Zweigteile sind beweglich zueinander mittels eines elektrisch isolierten Gelenks verbunden. In ihrem proximalen Ende sind die Zweigteile mit einem Mittel zur Steuerung der Zweigteilbewegung sowie mit einem Stromanschluss versehen. Am distalen Ende hat jedes der Zweigteile eine Verbundstruktur aus einem leitfähigen Teil und einem dielektrischen Teil. Es ist mit einer Schneidkante versehen. Dieses Schneidinstrument zeichnet sich dadurch aus, dass es für Gewebeeinschnitte mit einer Vorgerinnung angepasst ist und am distalen Ende der Zweigteile Folgendes enthält:
- einen dielektrischen Teil: er ist an der Innenseite des leitfähigen Teils angeordnet und als Klinge ausgebildet; die Klinge ist als Platte mit kleinem Trapez im Querschnitt ausgebildet; die Platte ist über ihre gesamte Länge mit einer Fase unter einem Winkel zur Schnittebene versehen; diese Fase stellt die Ausbildung der Schneidekante und der davon abstehenden Stumpfkante der Berührungslinie der Zweigteilklingen untereinander in der Schnittebene sicher; die Oberfläche der der Schnittebene zugewandten Klingenflanke ist von der Schnittebene abgeneigt;
- einen leitfähigen Teil: er ist mit einer Druckkante versehen; sie dient zum Kontakt mit dem Biogewebe, um die Verletzung des Biogewebes zu vermeiden; die Druckkante tritt in Bezug auf die genannte Schneidkante der Klinge um den Abstand h vor; dieser Abstand stellt je nach dem Öffnungswinkel α der Distalenden des Zweigteils die vorgegebene Länge L der Vorlaufkontakt-Zone der Druckkante mit dem Biogewebe bis zur Kontaktstelle der Schneidkante mit dem Biogewebe sicher; dieser Abstand wird durch folgende Formel festgelegt: h = *L* . sin 0,5α.

Dabei sind die genannten Klingen aus einem Stück Einkristall eines harten, rissbeständigen, abnutzungsfesten, porenfreien Materials mit Antihafteigenschaften auf der Basis des teilweise stabilisierten Zirkoniumdioxids gefertigt. Dabei wird das teilweise stabilisierte Zirkoniumdioxid mittels gerichteter Kristallisation der Zirkoniumdioxid-Schmelze hergestellt. Das Zirkoniumdioxid enthält eine stabilisierende Komponente. Bei einer Domänengröße von unter 0,2µm hat Zirkoniumdioxid ein Monoblock-Oberflächengefüge und weist eine Phasenhomogenität der Zusammensetzung in Bezug auf den Umfang und die Oberfläche mindestens im Bereich der Schneidkante auf.

Dabei ist es erfindungsgemäß zweckmäßig, dass die genannten Schneidkanten der genannten Klingen entlang einer der kristallographischen Achsen <100> des Einkristallgitters ausgerichtet ausgebildet sind.

Dabei ist es erfindungsgemäß zweckmäßig, dass die Schneidkanten der Klingen geradlinig ausgebildet werden.

Dabei kann als Stabilisationskomponente erfindungsgemäß Yttriumoxid verwendet werden.

Dabei können die genannten Klingen erfindungsgemäß aus einem Stück Einkristall des genannten, stabilisierten Zirkoniumdioxids gefertigt werden. Dabei wird das stabilisierte Zirkoniumdioxid aus der Schmelze von Zirkoniumdioxid mit einem Yttriumoxid-Anteil von 0,2-8,0 Mol-% produziert.

Dabei können die genannten Klingen erfindungsgemäß aus einem Stück Einkristall des genannten, stabilisierten Zirkoniumdioxids gefertigt werden. Das stabilisierte Zirkoniumdioxid wird aus der Schmelze von Zirkoniumdioxid mit einem bevorzugten Yttriumoxid-Anteil von 2,8-3,7 Mol-% hergestellt.

Dabei können die genannten Klingen erfindungsgemäß aus einem Stück Einkristall des genannten Zirkoniumdioxids gefertigt und mittels Oxiden der Seltenerdmetalle stabilisiert werden.. Dazu zählen Elemente von Zer bis Lutetium.

Dabei können die genannten Klingen erfindungsgemäß aus einem Stück Einkristall des genannten Zirkoniumdioxids gefertigt werden. Das Zirkoniumdioxid wird aus einer Schmelze produziert, die 0,1-5,0 Mol-% der stabilisierenden Oxide der Seltenerdmetalle enthält.

Dabei können die genannten Klingen erfindungsgemäß aus einem Stück Einkristall des genannten Zirkoniumdioxids gefertigt werden. Das Zirkoniumdioxid wird aus einer Schmelze hergestellt, die solche Beimischungen enthält, die den Färbungskontrast der Klinge im Hintergrund des Operationsfelds sicherstellen.

Dabei können die genannten Klingen erfindungsgemäß aus einem Stück Einkristall des genannten Zirkoniumdioxids gefertigt werden. Das Zirkoniumdioxid wird aus einer Schmelze produziert, die 0,1-5,0 Mol-% der stabilisierenden Oxide der Seltenerdmetalle enthält. Sie stellen den Färbungskontrast der Klinge im Hintergrund des Operationsfelds sicher.

Dabei ist es erfindungsgemäß zweckmäßig, dass die genannten Klingen aus einem Stück Einkristall des teilweise stabilisierten Einkristall-Zirkoniumdioxids gefertigt werden. Dieses Einkristallstück wird mittels der gerichteten Kristallisation der Schmelze hergestellt. Dafür wird die Schmelze in einem HF-Induktionsfeld von unten nach oben entlang der senkrechten Wachstumsachse mit einer Geschwindigkeit von 2-4mm/h im Laufe von 8-15 Stunden und danach mit einer Geschwindigkeit von 8-15mm/h im Laufe von 10-15 Stunden bewegt. Nachfolgend wird das Einkristall geglüht. Dieses Glühen stellt die Phasen- und die Gefügehomogenität seines Umfangs und seiner Oberfläche sicher.

Dabei ist es erfindungsgemäß zweckmäßig, dass die tetragonale Phasenhomogenität des Umfangs und der Oberfläche des Einkristalls sichergestellt wird.

Dabei kann die tetragonale Phasenhomogenität im Umfang und auf der Oberfläche des Einkristalls erfindungsgemäß durch ein Glühen des Einkristalls in der Luft bei einer Temperatur von 1250-1400°C im Laufe von 10-100 Stunden sichergestellt werden.

Dabei kann erfindungsgemäß die tetragonale Phasenhomogenität im Einkristallumfang und die Kontrastfärbung des Einkristalls mittels Glühen des Einkristalls bei einer Temperatur von 2000-2200°C in verdünnter Luft bei einem Druck von 10⁻²-10-⁴mm Hg im Laufe von 2-10 Stunden sichergestellt werden.

Dabei ist es erfindungsgemäß zweckmäßig, dass die genannten Klingen aus einem Stück Einkristall des teilweise stabilisierten Zirkoniumdioxids gefertigt werden. Das Zirkoniumdioxid enthält untereinander ausgerichtete Zwillingsgefüge unter einem Winkel von 45° zur Ebene der Verzwilligung Sie werden durch tetragonale Phasen mit unterschiedlichem Grad der Tetragonalität gebildet. Die Tetragonalitätsachsen der Zwillingsgefüge sind zueinander unter einem Winkel von 85-90° ausgerichtet und sind nicht kollinear. Die Klinge weist mindestens im Bereich der Schneidkante eine tetragonale Phasenhomogenität von Umfang und Oberfläche auf.

Dabei ist es erfindungsgemäß zweckmäßig, dass die genannten Klingen aus einem Stück Einkristall des teilweise stabilisierten Zirkoniumdioxids gefertigt werden. Das Zirkoniumdioxid enthält Zwillingsgefüge, die mittels Kristallen der Tetragonalphasen ausgebildet sind. Der Tetragonalitätsgrad beträgt 1,005-1,007 und 1,014-1,035.

Dabei kann die genannte, tetragonale Phasenhomogenität des Materials der Klingen erfindungsgemäß mittels eines Vor-Reduktionsglühen der Klingen mindestens im Bereich der Schneidkante bis zu ihrem Einsatz in den leitfähigen Teilen der Zweigteile sichergestellt werden.

Dabei ist es erfindungsgemäß zweckmäßig, dass die Klingen oder das komplette Instrument in der Luft geglüht wird. Dabei werden sie innerhalb von 2-5 Stunden bei einer Temperatur von 1200-1350°C bei einer Temperaturzunahmegeschwindigkeit von 6-10°C/min und einer Temperaturabnahmegeschwindigkeit von 6-8°C/min gehalten.

Dabei kann die Schneidkantenbreite erfindungsgemäß 0,2-0,5µm betragen.

Dabei ist es erfindungsgemäß zweckmäßig, dass die genannten Schneidkanten der Klingen über die gesamte Länge mit einer Fase unter einem Winkel von 1-2° zur Schnittebene versehen sind. Der Abstand der Schneidkante zur Schnittebene beträgt 0,5-10µm.

Außerdem sollte die genannte, der Schnittebene zugewandte Oberfläche der Klinge zweckmäßig erfindungsgemäß von der Schnittebene um den Winkel von 1-2° abgelenkt werden.

Dabei kann die Größe L der Vorlaufkontakt-Zone der Druckkante erfindungsgemäß 1-5mm betragen.

Dabei kann die Klingenstärke erfindungsgemäß 0,1-2,0mm betragen.

Dabei kann die Klinge erfindungsgemäß eine variable Stärke entlang der Schneidkante aufweisen.

Dabei können die Klingen erfindungsgemäß in Form von abnehmbaren Einsatzelementen ausgebildet werden.

Dabei können die Klingen erfindungsgemäß in Form von Laschen ausgebildet werden. Die Laschen werden auf der Oberfläche der leitfähigen Teile der Zweigteile angeordnet.

Dabei kann das elektrochirurgische, bipolare Schneidinstrument erfindungsgemäß als Instrument für laparoskopische Operationen ausgebildet werden.

Dabei kann das elektrochirurgische, bipolare Schneidinstrument erfindungsgemäß als Instrument für Endoskopie (Spiegelung) ausgebildet werden.

### Kurzbeschreibung der Zeichnungen

Nachfolgend wird die Erfindung anhand von Ausführungsbeispielen und der beiliegenden Zeichnungen näher erläutert. Es zeigen:
- Fig. 1a, 1b: eine elektronenmikroskopische Abbildung des Gefüges einer Einkristallprobe des teilweise stabilisierten Zirkoniumdioxids und die Ausrichtung der Probeebene (100),
- Fig. 2a, 2b: eine Klinge (1), erfindungsgemäß in Form eines Einsatzelements für die Anwendung in einer chirurgischen Schere und eine Ausgestaltungsform,
- Fig.3: eine Ausgestaltungsform einer chirurgischen Schere (8) gemäß der Erfindung,
- Fig. 4a, 4b, 4c: Funktionsschema der Klingen 1 in einer chirurgischen Schere: Fig. 4a die Position der Klingen (1) bis zum Schneiden, Fig.4b die Position der Klingen (1) beim Schneiden, Fig. 4c die Position der Klingen (1) bei geschlossener Schere,
- Fig. 5: elektrochirurgisches, bipolares Schneidinstrument, erfindungsgemäß mit Vorausgerinnung, Ausgestaltungsform als elektrochirurgische, bipolare Schere,
- Fig. 6: ein Schema für die Durchführung einer Gerinnung und Gewebedurchtrennung, Ausgestaltungsform für den distalen Teil, und
- Fig. 7: Ansicht der der Schnittebene zugewandten Zweigteilinnenfläche, Ausgestaltungsform.

Die angeführten Beispiele begrenzen dabei nicht die Ausführungsmöglichkeiten der Erfindung und gehen nicht über den Schutzumfang der Ansprüche hinaus.

### Beste Ausgestaltungsform der Erfindung

Die Klingen für ein chirurgisches Instrument können erfindungsgemäß aus einem Stück Einkristall des teilweise stabilisierten Zirkoniumdioxids gefertigt werden. Dabei wird das teilweise stabilisierte Zirkoniumdioxid mittels einer gerichteten Kristallisation der Zirkoniumdioxid-Schmelze hergestellt. Das stabilisierte Zirkoniumdioxid enthält eine stabilisierende Komponente in einem kalten Behälter. Dabei kann als stabilisierende Komponente Yttriumoxid in einer Menge von 0,2-8,0 Mol-% und vorzugsweise 2,8-3,7 Mol-% von Yttriumoxid oder 0,1-5,0 Mol-% von Seltenerdmetalloxiden von Zer bis Lutetium benutzt werden. Außerdem kann die Schmelze durch solche Beimischungen ergänzt werden, die die Kontrastfärbung des Klingenmaterials im Hintergrund des Operationsfelds sicherstellen. Mögliche Farben sind z.B.: milchweiß, rosig, lila, gelb, rot, orange, hellblau, grünlichgelb, violett, Elfenbein, nassasphaltgrau oder schwarz.

Die Kristallisation wird z.B. in einem Behälter durchgeführt. Auf dem Behälterboden ist eine wärmedämmende Schicht der Ausgangsmaterialien angeordnet. Die Ausgangsmaterialien werden im Umfang des Behälters als konzentrische Schichten gelegt. In der Mitte befindet sich das metallene Zirkonium, um das Anfangsschmelzen sicherzustellen. Das Abschmelzen der nachfolgenden Schichten wird durch das Schmelzen von Zirkonium ausgelöst. Dies erfolgt in einem HF-Induktionsfeld bei gleichzeitiger Abkühlung der Behälterwände und des Behälterbodens. Die resultierende Schmelze wird 3-4 Stunden lang gehalten. Danach wird eine gerichtete Kristallisation der Schmelze vorgenommen. Dafür wird die Schmelze im HF-Induktionsfeld abwärts entlang der senkrechten Wachstumsachse bewegt, und zwar mit einer Geschwindigkeit von 2-4mm/h im Laufe von 8-15 Stunden, und danach mit einer Geschwindigkeit von 8-15mm/h im Laufe von 10-15 Stunden.

Anschließend wird der Einkristall geglüht. Dies stellt die Phasenhomogenität in seinem Umfang sicher. Dabei kann das Glühen erfindungsgemäß in der Luft bei einer Temperatur von 1250-1400°C im Laufe von 10-100 Stunden oder in verdünnter Luft bei einer Temperatur von 2000-2200°C bei einem Druck von 10⁻²- 10⁻⁴ mm Hg im Laufe von 2-10 Stunden erfolgen (Im zweiten Fall wird die Farbe des Einkristalls geändert).

Das hergestellte Material weist bei einer Domänengröße von unter 0,2µm ein Monoblock-Oberflächengefüge und eine tetragonale Phasenhomogenität des Materials im Umfang auf. Es enthält Zwillingsgefüge, die miteinander unter einem Winkel von 45° zur Ebene der Verzwilligung ausgerichtet sind. Sie sind mittels der Kristalle der Tetragonalphasen mit Tetragonalitätsgraden von 1,005-1,007 und 1,014-1,035 ausgebildet. Ihre Tetragonalitätsachsen sind zueinander unter einem Winkel von 85-90° ausgerichtet und sind nicht kollinear.

Das resultierende Material weist eine Mikrohärte von 15,08 GPa, eine Biegefestigkeit von 800-1200 MPa, eine Bruchzähigkeit von 10 MPa*m^{0,5.} auf. Es ist ein porenfreies und abnutzungsfestes Dielektrikum.

Der Ablauf bei Klingenfertigung aus dem Einkristall des teilweise stabilisierten Zirkoniumdioxids umfasst einige Schritte:
1. Ausrichtung des Einkristalls gemäß den kristallographischen Achsen <100> des Gitters;
2. Schneiden des Einkristalls in Platten mit vorgegebenen Größen; dabei wird die Anordnung der Schneidkante im Einkristall mit der Ausrichtung entlang einer der kristallographischen Achsen <100> des Einkristallgitters sichergestellt;
3. die mechanische Bearbeitung umfasst die Maßbearbeitung, das Schleifen, da Bohren und den Anschliff.
   Dabei kann die Klinge entlang die Schneidkante eine konstante oder variable Stärke haben. Die Schneidkante kann geradlinig oder krummlinig ausgebildet werden. Sie kann unterschiedliche Anschliffwinkel haben. Die Schneidkante kann 0,2-0,5µm breit sein.
   Dabei werden die Klingen für die Anwendung in einer chirurgischen Schere oder bei elektrochirurgischen Schneidinstrumenten, z.B. in Instrumenten für Laparoskopie, Endoskopie (Spiegelung), in Form einer Lasche oder eines Einsatzelements ausgebildet. Sie werden z.B. in Form von Platten mit geringem Trapez im Querschnitt mit einer Schneidkante ausgebildet. Die Schneidkante ist über die gesamte Länge mit einer Fase unter einem Winkel zur vermutlichen Schnittebene, z.B. unter einem Winkel von 1-2° zur Schnittebene, versehen. Die Fase dient zur Ausbildung der Schneidkante und der davon z.B. 0,5-10µm abstehenden Stumpfkante der vermutlichen Berührung der Instrumentklingen untereinander in der Schnittebene. Dabei kann die der Schnittebene zugewandte Klingenoberfläche um 1-2° von der Schnittebene abgelenkt werden. Die Klingenstärke kann 0,1-2,0mm betragen.
   Die nach dem oben beschriebenen Verfahren hergestellten Abschnitte der Klingenoberfläche wurden erforscht. Die Forschungen haben Folgendes gezeigt: im Hintergrund der Hauptausrichtung [001] gibt es Abschnitte mit einer Senkrechten [110] auf der Oberfläche der Klinge. Das zeugt von einer vorliegenden Blockbildung auf der Oberfläche der Probe. Der Winkel der Fehlorientierung bei Nachbarblöcken beträgt 90°.
4. Die fertige Klinge wird geglüht, um die Rissbeständigkeit der Schneidkante zu erhöhen, und zwar dadurch, dass die während der mechanischen Bearbeitung entstandene, monokline Phase auf der Klingenoberfläche beseitigt und die tetragonale Phasenhomogenität mindestens im Bereich der Schneidkante wiederhergestellt wird.

Dabei kann die Klinge erfindungsgemäß in der Luft geglüht werden, einschließlich des Haltens innerhalb von 2-5 Stunden bei einer Temperatur von 1200-1350°C mit einer Temperaturzunahmegeschwindigkeit von 6-10°C/min und mit einer Temperaturabnahmegeschwindigkeit von 6-8°C/min.

Die Klingenoberflächengüte nach dem Glühen wurde nach dem Verfahren der Elektronenmikroskopie unter Einsatz der Diffraktion der rückgestrahlten Elektronen (EBSD) geprüft. Nach dem Glühen wies die Klingenoberfläche komplett ein Monoblock-Gefüge der Tetragonalphase auf.

Somit ermöglicht die Wärmebehandlung der aus dem teilweise stabilisierten Zirkoniumdioxid hergestellten Klingen, beim genannten Temperaturverlauf eine Oberfläche zu gewinnen, die eine hohe Gefüge- und Phasenhomogenität der Oberflächen bei Schneidkanten aufweisen. Die beschriebene Glüharbeit führt zur Erhöhung der Rissbeständigkeit der Schneidkante und beeinträchtigt nicht ihre Schärfe.

Aufgrund der fehlenden Mikroporosität sind die Antihafteigenschaften der Klingen aus dem teilweise stabilisierten Zirkoniumdioxid diesen der Keramik überlegen. Die Antihafteigenschaften legen die Dauer der Vorsterilisationsreinigung, z.B. mittels Behandlung im Ultraschallbad, fest. Die Verwendung der wärmebehandelten Klingen vermindert die Dauer der Vorsterilisationsreinigung um das 3-5-Fache im Vergleich zu Keramikmaterialien mit Korngröße von 1-5µm.

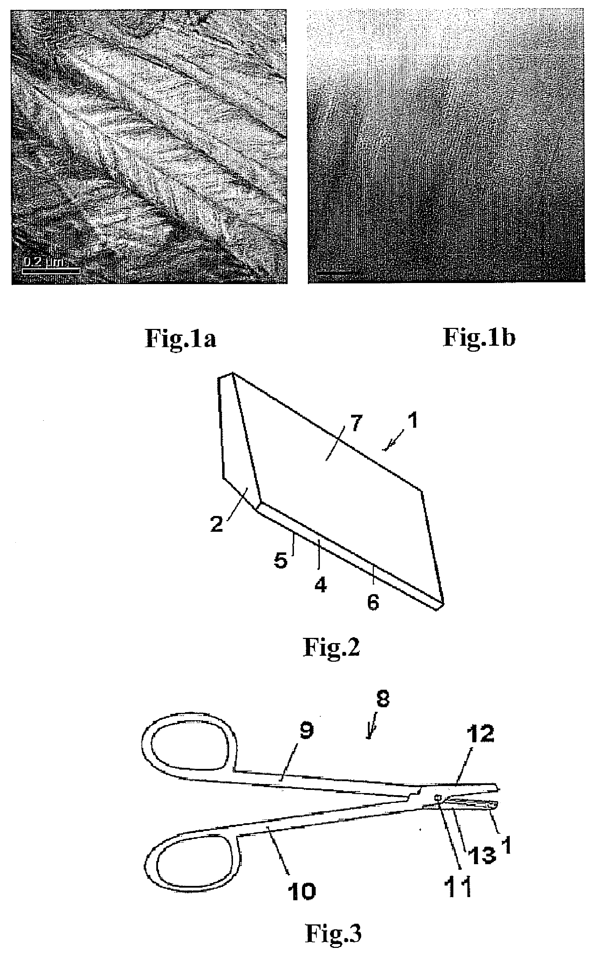

Die Fig. 1 zeigt die elektronenmikroskopische Abbildung des Gefüges der Einkristallprobe des teilweise stabilisierten Zirkoniumdioxids mit der Zusammensetzung ZrO₂ + 3,2 Mol-% Y₂O₃ und die Ausrichtung der Probeebene (100). Aus Fig. 1a ist ersichtlich, dass die meisten Domänen eine verlängerte Form aufweisen. Die primären Zwillingsplatten verzwilligen sich ihrerseits und bilden somit ein parkettartiges Gefüge aus Zwillingsdomänen. Die Spuren der sekundären Verzwilligungsebene liegen mit einem Winkle von etwa 45° zur Spur der Ebene der Primärverzwilligung. Die Fig. 1b zeigt das Ergebnis der elektronenmikroskopischen Untersuchung des Kristallprobegefüges bei hoher Auflösung, die bei etwa 10 nm breiten Mikrozwillingen vorhanden ist.

Die Fig. 2a, 2b zeigen erfindungsgemäß die Ausgestaltungsform der Klinge 1 für die Anwendung in Form eines Einsatzelements, z.B. eines abnehmbaren Einsatzelements, in der chirurgischen Schere der Fig. 3.

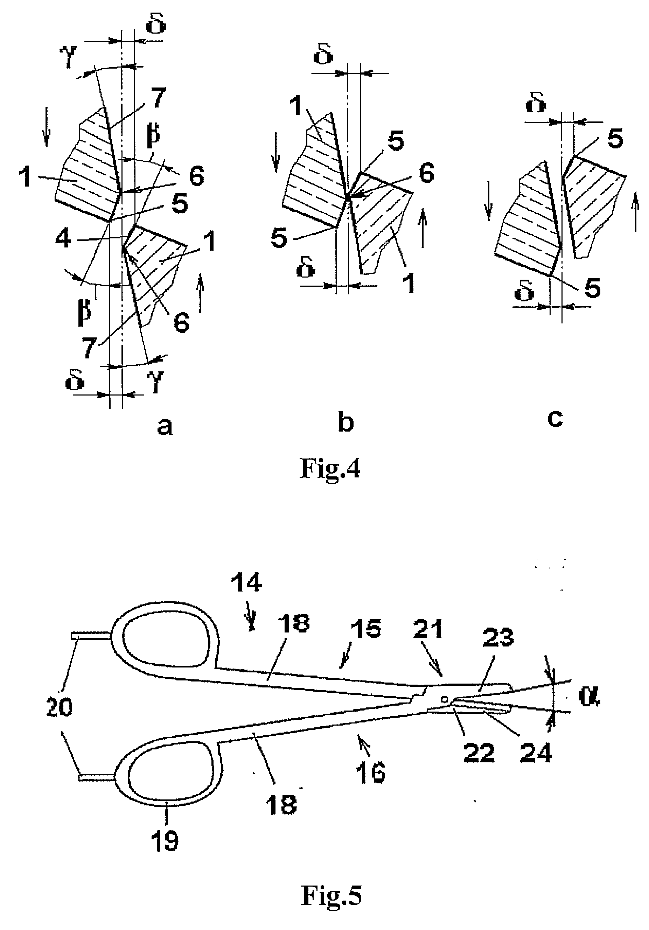

Die Fig. 4a, 4b, 4c enthalten das Funktionsschema der Klingen 1: Fig. 4a zeigt die Position der Klingen 1 bis zum Schneiden, die Fig. 4b zeigt die Position der Klingen 1 beim Schneiden, und die Fig. 4c zeigt die Position der Klingen 1 bei geschlossener Schere.

Die Klinge 1 (Fig. 2a, b) ist nach einem der oben beschriebenen Ausgestaltungsformen aus einem Stück Einkristall eines harten, rissbeständigen, abnutzungsfesten, porenfreien Materials mit Antihafteigenschaften auf der Basis des teilweise stabilisierten Einkristalls Zirkoniumdioxid ausgebildet. Dabei wird das teilweise stabilisierte Zirkoniumdioxid mittels gerichteter Kristallisation der Zirkoniumdioxid-Schmelze hergestellt. Das Zirkoniumdioxid enthält eine stabilisierende Komponente und weist bei einer Domänengröße von unter 0,2µm ein Monoblock-Oberflächengefüge und eine Phasenhomogenität der Zusammensetzung im Umfang mindestens im Bereich der Schneidekante auf. Die Ausbildung erfolgt z.B. aus einem Stück Einkristall des teilweise stabilisierten Zirkoniumdioxids. Das Zirkoniumdioxid enthält untereinander ausgerichtete Zwillingsgefüge unter einem Winkel von 45° zur Ebene der Verzwilligung. Sie werden durch tetragonale Phasen mit unterschiedlichem Grad der Tetragonalität ausgebildet, z.B. mit Tetragonalitätsgraden von 1,005-1.007 und 1,014-1,035. Ihre Tetragonalitätsachsen sind zueinander unter einem Winkel von 85-90° ausgerichtet und sind nicht kollinear.

Die Klinge 1 (Fig. 2a, b) ist als Platte 2 ausgebildet. Die Platte 2 ist über ihre gesamte Länge mit einer Fase 4 mit z.B. einem Winkel von β=1-2° zur vermutlichen Schnittebene 3 versehen. Die Fase 4 ist für die Ausbildung einer geradlinigen Schneidkante 5 und einer davon abstehenden Stumpfkante 6 der Berührungslinie der Instrumentklingen miteinander in der Schnittebene 3 vorgesehen. Dabei weist die Klinge 1 mindestens im Bereich der Schneidkante 5 eine tetragonale Phasenhomogenität des Umfangs und der Oberfläche auf. Die Schneidkante 5 der Klinge 1 ist entlang einer der kristallographischen Achsen <100> des Einkristallgitters ausgerichtet ausgebildet. Die der Schnittebene 3 zugewandte Oberfläche 7 der Klinge ist z.B. um einen Winkel von y=1-2° von der Schnittebene 3 abgelenkt. Der Abstand zwischen der Schneidkante 5 und der Schnittebene 3 beträgt ö=0,5-10µm. Die Klingenstärke (der Platte 2) beträgt 0,1-2,0mm. Die Güte des Klingenwerkstoffs ermöglicht es, 0,2-0,5µm breite Schneidkanten anzufertigen.

Die Fig. 3 zeigt eine Ausgestaltungsform der chirurgischen Schere 8 gemäß der Erfindung. Die Schere hat zwei Zweigteile 9 und 10. Die Zweigteile 9 und 10 sind beweglich miteinander mittels eines Gelenks 11 verbunden. An ihren distalen Enden 12 und 13 sind Einsatzelemente in Form von Klingen 1 in der oben beschriebenen Ausführung angeordnet. Beim Schneiden (Fig. 4a, 4b, 4c) wird nur der Kontakt der Stumpfkanten 6 beim Schließen der Zweigteile 9 und 10 im Schnittpunkt sichergestellt. Die Schneidkanten 5 berühren dagegen ausschließlich das Biogewebe. Das erhöht wesentlich die Abnutzungsfestigkeit der Schere.

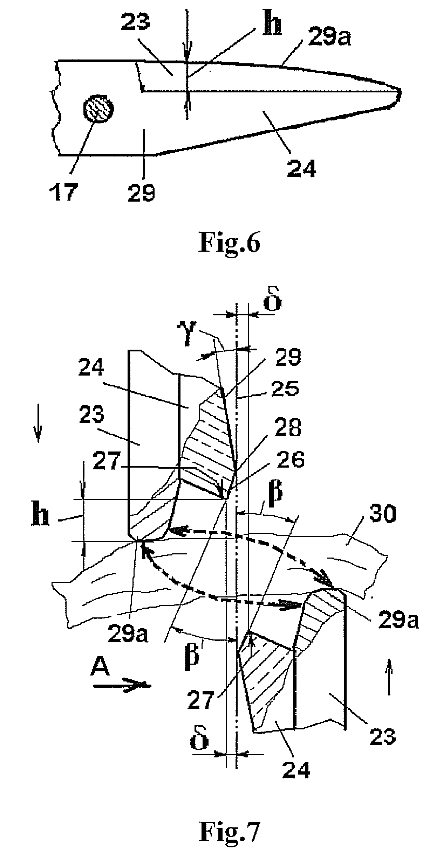

Die Fig. 5 zeigt eine Ausgestaltungsform des elektrochirurgischen, bipolaren Schneidinstruments in Form einer elektrochirurgischer, bipolarer Schere gemäß der Erfindung. Die Fig. 6 zeigt ein Schema für die Durchführung der Gerinnung und Gewebedurchtrennung. In Fig. 7 ist die Zweigteilinnenfläche abgebildet, die der Schnittebene zugewandt ist.

Das elektrochirurgische, bipolare Schneidinstrument 14 (Fig. 5, 6, 7) ist erfindungsgemäß für Gewebeeinschnitte mit einer Vorgerinnungswirkung angepasst. Das Instrument enthält zwei isolierte, leitfähige Zweigteile 15 und 16. Die Zweigteile sind beweglich zueinander mittels eines elektrisch isolierten Gelenks 17 verbunden. An ihrem proximalen Ende 18 sind sie mit einem Mittel zur Zweigteilsteuerung versehen. Das können z.B. Ringe 19 sein. Die Zweigteile sind auch mit einem Anschluss 20 für die Stromversorgung versehen. Am Distalende 21 jedes Zweigteils 15, 16 ist ein dielektrischer Teil 22 und ein leitfähiger Teil 23 angeordnet.

Der dielektrische Teil 22 ist in Form einer Klinge 24 ausgebildet, die an der Innenseite des leitfähigen Teils 23 angeordnet wird. Die Klinge 24 ist als Lasche nach einer der oben beschriebenen Ausgestaltungsformen aus einem Stück Einkristall des harten, rissbeständigen, abnutzungsfesten, porenfreien Materials mit Antihafteigenschaften auf der Basis des teilweisen stabilisierten Zirkoniumdioxids gebildet. Dabei wird das teilweise stabilisierte Zirkoniumdioxid mittels gerichteter Kristallisation der Zirkoniumdioxid-Schmelze hergestellt. Das Zirkoniumdioxid enthält eine stabilisierende Komponente und weist bei einer Domänengröße von unter 0,2µm ein Monoblock-Oberflächengefüge und eine Phasenhomogenität der Zusammensetzung im Umfang mindestens im Bereich der Schneidkante auf. Die Klingen sind z.B. aus einem Stück Einkristall des teilweise stabilisierten Zirkoniumdioxids gefertigt. Das Zirkoniumdioxid enthält untereinander ausgerichtete Zwillingsgefüge unter einem Winkel von 45° zur Verzwilligungsebene. Sie werden durch tetragonale Phasen mit einem unterschiedlichen Grad der Tetragonalität ausgebildet, z.B. mit Tetragonalitätsgraden von 1,005-1,007 und 1,014-1,035. Ihre Tetragonalitätsachsen sind zueinander unter einem Winkel von 85-90° ausgerichtet und sind nicht kollinear.

Die Klingen 24 (Fig. 5, 6 ,7) sind als Platten ausgebildet. Die Platte ist über ihre gesamte Länge mit einer Fase 26 schräg, z.B. mit einem Winkel von β=1-2° zur vermutlichen Schnittebene 25, versehen. Die Fase 25 stellt die Ausbildung der geradlinigen Schneidkante 27 und der davon abstehenden Stumpfkante 28 der Berührungslinie der Klingen 24 des Instruments 14 miteinander in der Schnittebene 3 sicher. Dabei weist die Klinge 24 mindestens im Bereich der Schneidkante 27 eine tetragonale Phasenhomogenität des Umfangs und der Oberfläche auf. Die Schneidkante 27 der Klinge 24 ist mit der Ausrichtung entlang eine der kristallographischen Achsen <100> des Einkristallgitters ausgebildet. Die der Schnittebene 25 zugewandte Oberfläche 29 der Klinge ist von der Schnittebene 25 um z.B. einen Winkel von **y**=1-2° abgelenkt. Der Abstand zwischen der Schneidkante 27 und der Schnittebene 25 beträgt ö=0,5-10µm. Die Klingenstärke (Platte) beträgt 0,1-2,0mm. Die Güte des Klingenmaterials 24 ermöglicht es, die Schneidkante mit einer Breite von 0,2-0,5µm auszubilden.

Der leitfähige Teil 23 (Fig. 5, 6, 7) ist mit einer Druckkante 29 versehen. Sie ist für einen Kontakt mit dem Biogewebe 30 angepasst. Er schließt die Verletzung des Biogewebes 30 aus. Die Druckkante 29 ist so ausgebildet, dass sie um einen Abstand h zur Schneidkante 27 der Klinge 24 vortritt. Der Abstand h stellt je nach Öffnungswinkel α der distalen Enden 21 der Zweigteile 15, 16 die vorgegebene Größe L der Vorlaufkontakt-Zone der Druckkante 29 mit dem Biogewebe 30 bis zur Kontaktstelle der Schneidkante 27 mit dem Biogewebe 30 sicher. Dieser Abstand wird nach der Formel h=L.sin0,5α ermittelt. Die Größe L kann aus dem Bereich von 1-5mm gewählt werden.

Dabei können die Schneidkanten 27 der Klingen 24 geradlinig oder krummlinig je nach gewählter Gestaltung der Zweigteile ausgebildet werden. Dabei hängt die Quergröße L des Gerinnungsbereichs von der Stärke der Klinge 24 ab. Diese Stärke kann sowohl konstant als auch variabel entlang der Schneidkante 27 sein. Sie kann im Bereich von z.B. 0,1- 2,0mm liegen.

Dabei können die Klingen 24 als einteilige oder abnehmbare Einsatzelemente oder Laschen ausgebildet werden.

Die Zweigteile dienen als Elektroden. Wenn der Strom an die leitfähigen Teile 16 der Zweigteile angelegt wird, so löst der durch das Biogewebe über die kürzeste Strecke zwischen den Druckkanten 28 fließende HF-Strom die Gerinnung der Biogewebe aus. Wenn die distalen Enden 15 und Klingen 24 einander entgegen kommen, erfolgt eine mechanische Durchtrennung des Biogewebes 30 mittels der Schneidkanten 27 der Klingen 24. Die Klingen 24 kommen nur an der Berührungsstelle der Stumpfkanten 28 in Kontakt miteinander. Dabei sind die Vorgänge, wie die Durchtrennung und die Koagulation des biologischen Gewebes in der Zeit und im Raum auseinander gesetzt. Die der Phase des mechanischen Schnitts vorlaufende Gerinnungsphase stellt das Fehlen von Blutungen (optimale Blutstillung) während des chirurgischen Schnitts von biologischen Geweben sicher. Die Klingen 24 berühren einander nicht über die gesamte Seitenfläche der Klinge 24, sondern nur an einer Stelle der Stumpfkante 28. Das stellt eine Erhöhung der Abnutzungsfestigkeit des Klingenmaterials sicher.

Somit ermöglichen die einzigartigen Eigenschaften der Klingen gemäß der Erfindung, hohe Leistungsmerkmale und neue Möglichkeiten der zuverlässigen und benutzerfreundlichen, chirurgischen Instrumente sicherzustellen, einschließlich der chirurgischen Schere und der elektrochirurgischen, bipolaren Schneidinstrumente zur Durchtrennung von Biogewebe mit Vorgerinnung. Die Klingen und Instrumente gemäß der Erfindung können unter Inanspruchnahme der bekannten und oben beschriebenen Technologien ausgebildet werden.

## Patentansprüche

1. Klinge für ein chirurgisches Instrument, die aus einem dielektrischem Material gebildet und mit einer Schneidkante versehen ist,
**dadurch gekennzeichnet,**
**dass** die Klinge aus einem Stück Einkristall eines harten, rissbeständigen, abnutzungsfesten, porenfreien Materials mit Antihafteigenschaften auf der Basis von teilweise stabilisierten Zirkoniumdioxids gefertigt wird, wobei das teilweise stabilisierte Zirkoniumdioxid mittels einer gerichteten Kristallisation der Zirkoniumdioxid-Schmelze hergestellt wird und das Zirkoniumdioxid eine stabilisierende Komponente enthält sowie bei einer Domänengröße von unter 0,2µm ein Monoblock-Oberflächengefüge, eine Phasenhomogenität der Zusammensetzung in Bezug auf den Umfang und die Oberfläche mindestens im Bereich der Schneidkante aufweist.

2. Klinge nach Anspruch 1,
**dadurch gekennzeichnet,**
**dass** die Schneidkante der genannten Klinge mit der Ausrichtung entlang einer der kristallographischen Achsen (100) des Einkristallgitters ausgebildet ist.

3. Klinge nach Anspruch 1,
**dadurch gekennzeichnet,**
**dass** sie aus einem Stück Einkristall des stabilisierten Zirkoniumdioxids gefertigt ist,
**dass** dieses stabilisierte Zirkoniumdioxid mittels einer gerichteten Kristallisation der Schmelze hergestellt wird, indem die Schmelze in einem elektromagnetischen HF-Feld von unten nach oben entlang der senkrechten Wachstumsachse des Blocks mit einer Geschwindigkeit von 2-4mm/h im Laufe von 8-15 Stunden und danach mit einer Geschwindigkeit von 8-15 mm/h im Laufe von 10-15 Stunden bewegt wird, und
**dass** danach das Glühen des Einkristalls vorgenommen wird, um die Phasen- und die Gefügehomogenität seines Umfangs und seiner Oberfläche sicherzustellen.

4. Klinge nach Anspruch 1,
**dadurch gekennzeichnet,**
**dass** die tetragonale Phasenhomogenität im Umfang und auf der Oberfläche des Einkristalls sichergestellt wird.

5. Klinge nach Anspruch 1,
**dadurch gekennzeichnet,**
**dass** die genannte, tetragonale Phasenhomogenität im Umfang und auf der Oberfläche des Einkristalls durch das Glühen des Einkristalls in der Luft bei einer Temperatur von 1250-1400°C im Laufe von 10-100 Stunden sichergestellt wird.

6. Klinge nach Anspruch 1,
**dadurch gekennzeichnet,**
**dass** die tetragonale Phasenhomogenität im Einkristallumfang und die Kontrastfärbung des Einkristalls mittels Glühen des Einkristalls bei einer Temperatur von 2000-2200°C in verdünnter Luft bei einem Druck ab 10⁻²- 10⁻⁴mm Hg im Laufe von 2-10 Stunden sichergestellt wird.

7. Klinge nach Anspruch 1,
**dadurch gekennzeichnet,**
**dass** Yttriumoxid als Stabilisationskomponente wirkt.

8. Klinge nach Anspruch 6,
**dadurch gekennzeichnet,**
**dass** sie aus einem Stück Einkristall des genannten, stabilisierten Zirkoniumdioxids gefertigt wird, wobei das stabilisierte Zirkoniumdioxid aus der Schmelze von Zirkoniumdioxid mit einem Yttriumoxid-Anteil von 0,2-8,0 Mol-% produziert wird.

9. Klinge nach Anspruch 1,
**dadurch gekennzeichnet,**
**dass** sie aus einem Stück Einkristall des genannten Zirkoniumdioxids, stabilisiert mittels Oxiden der Seltenerdmetalle, gefertigt wird, wobei die Seltenerdmetalle Elemente von Zer bis Lutetium einschließen.

10. Klinge nach Anspruch 9,
**dadurch gekennzeichnet,**
**dass** sie aus einem Stück Einkristall des genannten Zirkoniumdioxids gefertigt wird und dass das Zirkoniumdioxid aus einer der Schmelze hergestellt wird, die 0,1-5,0 Mol-% von stabilisierenden Oxiden der Seltenerdmetalle enthält.

11. Klinge nach Anspruch 1,
**dadurch gekennzeichnet,**
**dass** sie aus einem Stück Einkristall des genannten Zirkoniumdioxids gefertigt wird und dass das Zirkoniumdioxid aus einer Schmelze hergestellt wird, die solche Beimischungen enthält, die den Färbungskontrast der Klinge im Hintergrund des Operationsfelds sicherstellen.

12. Klinge nach Anspruch 1,
**dadurch gekennzeichnet,**
**dass** sie aus einem Stück Einkristall des genannten Zirkoniumdioxids gefertigt wird, wobei das Zirkoniumdioxid aus einer Schmelze hergestellt wird, die 0,1-5,0 Mol-% von stabilisierenden Oxiden der Seltenerdmetalle enthält, die die Kontrastfärbung des Klingenmaterials im Hintergrund des Operationsfelds sicherstellen.

13. Klinge nach Anspruch 1,
**dadurch gekennzeichnet,**
**dass** sie aus einem Stück Einkristall des stabilisierten Zirkoniumdioxids gefertigt wird, wobei das Zirkoniumdioxid untereinander ausgerichtete Zwillingsgefüge unter einem Winkel von 45° zur Ebene der Verzwilligung enthält und die Zwillingsgefüge durch tetragonale Phasen mit einem unterschiedlichen Grad der Tetragonalität ausgebildet werden, wobei die Tetragonalitätsachsen zueinander unter einem Winkel von 85-90° ausgerichtet und nicht kollinear sind, und dass die Klinge mindestens im Bereich der Schneidkante eine tetragonale Phasenhomogenität aufweist.

14. Klinge nach Anspruch 13, d
**dadurch gekennzeichnet,**
**dass** sie aus einem Stück Einkristall des stabilisierten Zirkoniumdioxids hergestellt wird, dass das Zirkoniumdioxid Zwillingsgefüge enthält, die mittels Kristallen der Tetragonalphasen ausgebildet sind und dass der Tetragonalitätsgrad 1,005-1,007 und 1,014-1,035 beträgt.

15. Klinge nach Anspruch 13,
**dadurch gekennzeichnet,**
**dass** sie aus einem Stück Einkristall des genannten, stabilisierten Zirkoniumdioxids gefertigt wird und dass dieses stabilisierte Zirkoniumdioxid aus einer Zirkoniumdioxidschmelze mit 2,8-3,7 Mol-% Yttriumoxid hergestellt wird.

16. Klinge nach Anspruch 13, d
**dadurch gekennzeichnet,**
**dass** die genannte, tetragonale Phasenhomogenität mittels Glühen der fertigen Klinge mindestens im Bereich der Schneidkante der fertigen Klinge sichergestellt wird.

17. Klinge nach Anspruch 16,
**dadurch gekennzeichnet,**
**dass** sie in der Luft geglüht wird, einschließlich des Haltens im Laufe von 2-5 Stunden bei einer Temperatur von 1200-1350°C mit einer Temperaturzunahmegeschwindigkeit von 6-10°C/min und mit einer Temperaturabnahmegeschwindigkeit von *6-8°C*/*min.*

18. Klinge nach Anspruch 1,
**dadurch gekennzeichnet,**
**dass** die Schneidkante 0,2-0,5µm breit ist.

19. Klinge nach Anspruch 1,
**dadurch gekennzeichnet,**
**dass** die Schneidkante geradlinig ausgebildet ist.

20. Klinge nach Anspruch 1,
**dadurch gekennzeichnet,**
**dass** sie für die Anwendung in Form einer Lasche oder eines Einsatzelements in chirurgischen Schneidinstrumenten, vorzugsweise indarunter Skalpells, Schneidmessern, Scheren, elektrochirurgischen, unipolaren Schneidinstrumenten, elektrochirurgischen, bipolaren Schneidinstrumenten, angepasst ist.

21. Klinge nach Anspruch 20,
**dadurch gekennzeichnet,**
**dass** sie für die Anwendung in Form eines Einsatzelements für chirurgische Scheren angepasst wird und als Platte ausgebildet ist, dass die Platte über ihre gesamte Länge mit einer Fase geneigt zur vermutlichen Schneidebene versehen wird und dass die Fase die Ausbildung der Schneidkante und einer davon abstehenden Stumpfkante der Berührungslinie der Instrumentklingen untereinander in der Schnittebene sicherstellt.

22. Klinge nach Anspruch 20,
**dadurch gekennzeichnet,**
**dass** sie für die Anwendung in einem elektrochirurgischen, bipolaren Schneidinstrument angepasst und in Form einer Platte ausgebildet ist, dass die Platte über ihre gesamte Länge mit einer Fase geneigt zur vermutlichen Schneideebene versehen wird und dass die Fase die Ausbildung der Schneidkante und der davon abstehenden Stumpfkante der Berührungslinie der Instrumentklingen untereinander in der Schnittebene sicherstellt.

23. Chirurgische Schere mit zwei Zweigteilen, wobei die Zweigteile beweglich mittels eines Gelenks verbunden sind und wobei an ihren distalen Enden die Klingen mit Schneidkanten ausgebildet sind,
**dadurch gekennzeichnet,**
**dass** die Schere mit nach Anspruch 20 ausgebildeten Klingen versehen ist.

24. Elektrochirurgisches, bipolares Schneidinstrument mit zwei isolierten, leitfähigen Zweigteilen, die zueinander mittels eines elektrisch isolierten Gelenks beweglich verbunden sind und an ihrem proximalen Ende mit einem Mittel zur Steuerung der Zweigteilbewegung und mit einem Anschluss versehen ist, wobei jedes der Zweigteile an ihrem distalen Ende eine Verbundstruktur aus einem leitfähigen Teil und einem dielektrischen Teil aufweist und mit einer Schneidkante versehen ist,
**dadurch gekennzeichnet,**
**dass** dieses Schneidinstrument für Gewebeeinschnitte mit einer Vorgerinnung angepasst ist und am distalen Ende der Zweigteile Folgendes aufweist:
- den dielektrischen Teil, der an der Innenseite des leitfähigen Teils angeordnet und als Klinge ausgebildet ist, wobei die Klinge als Platte mit kleinem Trapez im Querschnitt ausgebildet ist und die Platte über ihre gesamte Länge mit einer Fase unter einem Winkel zur Schnittebene versehen ist, wobei die Fase die Ausbildung der Schneidkante und der davon abstehenden Stumpfkante der Berührungslinie der Branchenklingen untereinander in der Schnittebene sicherstellt und die Oberfläche der der Schnittebene zugewandten Klingenflanke von der Schnittebene abgelenkt ist,
- den leitfähigen Teil, der mit einer Druckkante versehen ist, die zum Kontakt mit dem Biogewebe dient, um die Verletzung von Biogewebe zu vermeiden, wobei die Druckkante in Bezug auf die genannte Schneidkante der Klinge um einen Abstand *h* vortritt und dieser Abstand je nach dem Öffnungswinkel α der Distalenden der Zweigteile eine vorgegebene Länge L der Vorlaufkontakt-Zone der Druckkante mit dem Biogewebe bis zur Kontaktstelle zwischen der Schneidkante und dem Biogewebe sicherstellt sowie die Länge L mit Hilfe der folgenden Formel h=L-sin0,5α ermittelt wird,
wobei die genannten Klingen aus einem Stück Einkristall eines harten, rissbeständigen, abnutzungsfesten, porenfreien Materials mit Antihafteigenschaften auf der Basis des teilweise stabilisierten Zirkoniumdioxids gefertigt sind und das teilweise stabilisierte Zirkoniumdioxid mittels gerichteter Kristallisation der Zirkoniumdioxid-Schmelze hergestellt wird, und dass die Zirkoniumdioxid-Schmelze enthält eine stabilisierende Komponente enthält und bei einer Domänengröße von unter 0,2µm ein Monoblock-Oberflächengefüge und eine Phasenhomogenität der Zusammensetzung in Bezug auf den Umfang und die Oberfläche mindestens im Bereich der Schneidkante aufweist.

25. Instrument nach Anspruch 24,
**dadurch gekennzeichnet,**
**dass** die genannten Schneidkanten der genannten Klingen entlang einer der kristallographischen Achsen (100) des Einkristallgitters ausgerichtet sind.

26. Instrument nach Anspruch 24,
**dadurch gekennzeichnet,**
**dass** die Schneidkanten der Klingen geradlinig ausgebildet werden.

27. Instrument nach Anspruch 24,
**dadurch gekennzeichnet,**
**dass** als Stabilisationskomponente Yttriumoxid benutzt wird.

28. Instrument nach Anspruch 24,
**dadurch gekennzeichnet,**
**dass** die genannten Klingen aus einem Stück Einkristall des genannten, stabilisierten Zirkoniumdioxids gefertigt werden, wobei das stabilisierte Zirkoniumdioxid aus einer Schmelze von Zirkoniumdioxid mit einem Yttriumoxid-Anteil von 0,2-8,0 Mol-% hergestellt wird..

29. Instrument nach Anspruch 24, d
**dadurch gekennzeichnet,**
**dass** die genannten Klingen aus einem Stück Einkristall des genannten, stabilisierten Zirkoniumdioxids gefertigt werden, wobei das stabilisierte Zirkoniumdioxid aus einer Schmelze von Zirkoniumdioxid mit einem bevorzugten Yttriumoxid-Anteil von 2,8-3,7 Mol-% hergestellt wird.

30. Instrument nach Anspruch 24,
**dadurch gekennzeichnet,**
**dass** die genannten Klingen aus einem Stück Einkristall des genannten Zirkoniumdioxids gefertigt werden, das mittels Oxiden der Seltenerdmetalle stabilisiert wird, und dass als Seltenerdmetalle Elemente von Zer bis Lutetium verwendet werden.

31. Instrument nach Anspruch 30,
**dadurch gekennzeichnet,**
**dass** die genannten Klingen aus einem Stück Einkristall des genannten Zirkoniumdioxids gefertigt werden, wobei das Zirkoniumdioxid aus einer Schmelze hergestellt wird, die 0,1-5,0 Mol-% von stabilisierenden Oxiden der Seltenerdmetalle enthält.

32. Instrument nach Anspruch 24,
**dadurch gekennzeichnet,**
**dass** die genannten Klingen aus einem Stück Einkristall des genannten Zirkoniumdioxids gefertigt werden, wobei das Zirkoniumdioxid aus einer Schmelze hergestellt wird, die solche Beimischungen enthält, die den Färbungskontrast der Klinge im Hintergrund des Operationsfelds sicherstellen.

33. Instrument nach Anspruch 24,
**dadurch gekennzeichnet,**
**dass** die genannten Klingen aus einem Stück Einkristall des genannten Zirkoniumdioxids gefertigt werden, wobei das Zirkoniumdioxid aus einer Schmelze hergestellt wird, die 0,1-5,0 Mol-% von stabilisierenden Oxiden der Seltenerdmetalle enthält, und wobei die Seltenerdmetalle den Färbungskontrast der Klinge im Hintergrund des Operationsfelds sicherstellen.

34. Instrument nach Anspruch 24,
**dadurch gekennzeichnet,**
**dass** die genannten Klingen aus einem Stück Einkristall des teilweise stabilisierten Einkristall-Zirkoniumdioxids hergestellt werden, wobei dieses Einkristallstück mittels einer gerichteten Kristallisation der Schmelze hergestellt wird, dass die Schmelze dafür in einem HF-Induktionsfeld von unten nach oben entlang der senkrechten Wachstumsachse des Blocks bewegt wird, und zwar mit einer Geschwindigkeit von 2-4mm/h im Laufe von 8-15 Stunden und danach mit einer Geschwindigkeit von 8-15mm/h im Laufe von 10-15 Stunden, und dass das Einkristall nachfolgend geglüht wird, um die Phasen- und die Gefügehomogenität seines Umfangs und seiner Oberfläche sicherzustellen.

35. Instrument nach Anspruch 24,
**dadurch gekennzeichnet,**
**dass** die tetragonale Phasenhomogenität im Umfang und auf der Oberfläche des Einkristalls sichergestellt wird.

36. Instrument nach Anspruch 35,
**dadurch gekennzeichnet,**
**dass** die tetragonale Phasenhomogenität im Umfang und auf der Oberfläche des Einkristalls durch das Glühen des Einkristalls in der Luft bei einer Temperatur von 1250-1400°C im Laufe von 10-100 Stunden sichergestellt wird.

37. Instrument nach Anspruch 24,
**dadurch gekennzeichnet,**
**dass** die tetragonale Phasenhomogenität im Einkristallumfang und die Kontrastfärbung des Einkristalls mittels Glühen des Einkristalls bei einer Temperatur von 2000-2200°C in verdünnter Luft bei einem Druck ab 10-²-10⁻⁴mm Hg im Laufe von 2-10 Stunden sichergestellt wird.

38. Instrument nach Anspruch 24,
**dadurch gekennzeichnet,**
**dass** die genannten Klingen aus einem Stück Einkristall des teilweise stabilisierten Zirkoniumdioxids gefertigt werden, dass das Zirkoniumdioxid untereinander ausgerichtete Zwillingsgefüge unter einem Winkel von 45° zur Ebene der Verzwilligung enthält, dass die Zwillingsgefüge durch tetragonale Phasen mit einem unterschiedlichen Grad der Tetragonalität ausgebildet werden, dass die Tetragonalitätsachsen zueinander unter einem Winkel von 85-90° ausgerichtet und nicht kollinear sind und dass die Klinge mindestens im Bereich der Schneidkante eine tetragonale Phasenhomogenität von Umfang und Oberfläche aufweist.

39. Instrument nach Anspruch 38,
**dadurch gekennzeichnet,**
**dass** die genannten Klingen aus einem Stück Einkristall des teilweise stabilisierten Zirkoniumdioxids hergestellt werden und dass das Zirkoniumdioxid Zwillingsgefüge enthält, die mittels Kristallen der Tetragonalphasen ausgebildet sind, wobei derTetragonalitätsgrad 1,005-1,007 und 1,014-1,035 beträgt.

40. Instrument nach Anspruch 38,
**dadurch gekennzeichnet,**
**dass** die genannte, tetragonale Phasenhomogenität des Klingenmaterials mittels eines Vor-Reduktionsglühens der Klingen mindestens im Bereich der Schneidkante bis zu ihrem Einsatz in den leitfähigen Teilen der Zweigteile sichergestellt wird.

41. Instrument nach Anspruch 39,
**dadurch gekennzeichnet,**
**dass** die Klingen oder das komplette Instrument in der Luft geglüht werden bzw. wird, wobei sie 2-5 Stunden lang bei einer Temperatur von 1200-1350°C mit einer Temperaturzunahmegeschwindigkeit von 6-10°C/min und mit einer Temperaturabnahmegeschwindigkeit von 6-8°C/min gehalten werden.

42. Instrument nach Anspruch 24,
**dadurch gekennzeichnet,**
**dass** die Schneidkantenbreite 0,2-0,5µm beträgt.

43. Instrument nach Anspruch 24,
**dadurch gekennzeichnet,**
**dass** die genannten Schneidkanten der Klingen über die gesamte Länge mit einer Fase unter einem Winkel von 1-2° zur Schnittebene versehen sind und dass der Abstand der Schneidkante zur Schnittebene 0,5-1 0µm beträgt.

44. Instrument nach Anspruch 24,
**dadurch gekennzeichnet,**
**dass** die genannte, der Schnittebene zugewandte Oberfläche der Klinge um den Winkel von 1-2° von der Schnittebene abgelenkt ist.

45. Instrument nach Anspruch 24,
**dadurch gekennzeichnet,**
**dass** die Größe L der Vorlaufkontakt-Zone der Druckkante 1-5mm beträgt.

46. Instrument nach Anspruch 24,
**dadurch gekennzeichnet,**
**dass** die Klingenstärke 0,1-2,0mm beträgt.

47. Instrument nach Anspruch 24,
**dadurch gekennzeichnet,**
**dass** die Klinge eine variable Stärke entlang der Schneidkante aufweist.

48. Instrument nach Anspruch 24,
**dadurch gekennzeichnet,**
**dass** die Klingen als abnehmbare Einsatzelemente ausgebildet sind.

49. Instrument nach Anspruch 24,
**dadurch gekennzeichnet,**
**dass** die Klinge in Form einer Lasche ausgebildet wird, die an der Oberfläche der leitfähigen Teile der Zweigteile angeordnet wird.
